(19) Europäisches Patentamt
European Patent Office
Office européen des brevets

(11) EP 0 678 086 B1

(12) # EUROPEAN PATENT SPECIFICATION

(45) Date of publication and mention of the grant of the patent:
**08.12.1999 Bulletin 1999/49**

(21) Application number: **94901195.1**

(22) Date of filing: **22.10.1993**

(51) Int Cl.[6]: **C07C 65/36**, A61K 31/19, C07C 65/17, C07C 63/66, C07C 65/40, C07C 63/49

(86) International application number:
**PCT/US93/10204**

(87) International publication number:
**WO 94/15902 (21.07.1994 Gazette 1994/17)**

(54) **COMPOUNDS HAVING SELECTIVITY FOR RETINOID X RECEPTORS**

SELEKTIVITAET FUER RETINOID X REZEPTOREN ENTHALTENDE VERBINDUNGEN

COMPOSES A SELECTIVITE ENVERS LES RECEPTEURS DE RETINOIDES X

(84) Designated Contracting States:
**AT BE CH DE DK ES FR GB GR IE IT LI LU MC NL PT SE**

(30) Priority: **11.01.1993 US 3223**
**05.03.1993 US 27747**
**21.04.1993 US 52051**

(43) Date of publication of application:
**25.10.1995 Bulletin 1995/43**

(73) Proprietor: **LIGAND PHARMACEUTICALS, INC.**
**San Diego, CA 92121 (US)**

(72) Inventors:
- **BOEHM, Marcus, F.**
  **San Diego, CA 92109 (US)**
- **HEYMAN, Richard, A.**
  **Encinitas, CA 92024 (US)**
- **ZHI, Lin**
  **San Diego, CA 92129 (US)**
- **KOCH, Stacie, Canan**
  **San Diego, CA 92116 (US)**

(74) Representative: **Irvine, Jonquil Claire et al**
**J.A. KEMP & CO.**
**14 South Square**
**Gray's Inn**
**London WC1R 5LX (GB)**

(56) References cited:
**EP-A- 0 260 162** **GB-A- 2 197 316**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 678 086 B1

## Description

This invention relates to intracellular receptors and ligands therefor. More specifically, this invention relates to compounds having selective activity for specific retinoic acid receptors, and methods for use of such compounds.

## BACKGROUND OF THE INVENTION

The vitamin A metabolite retinoic acid has long been recognized to induce a broad spectrum of biological effects. A variety of structural analogues of retinoic acid have been synthesized that also have been found to be bioactive. Some, such as Retin-A® (registered trademark of Johnson & Johnson) and Accutane® (registered trademark of Hoffmann-LaRoche), have found utility as therapeutic agents for the treatment of various pathological conditions. Metabolites of vitamin A and their synthetic analogues are collectively herein called "retinoids".

Synthetic retinoids have been found to mimic many of the pharmacological actions of retinoic acid. However, the broad spectrum of pharmacological actions of retinoic acid is not reproduced in full by all bioactive synthetic retinoids.

Medical professionals have become very interested in the medicinal applications of retinoids. Among their uses approved by the FDA is the treatment of severe forms of acne and psoriasis. A large body of evidence also exists that these compounds can be used to arrest and, to an extent, reverse the effects of skin damage arising from prolonged exposure to the sun. Other evidence exists that these compounds may be useful in the treatments of a variety of severe cancers including melanoma, cervical cancer, some forms of leukemia, and basal and squamous cell carcinomas. Retinoids have also shown an ability to be efficacious in treating premalignant cell lesions, such as oral leukoplakia, and to prevent the occurrence of malignancy.

Use of the retinoids is associated with a number of significant side effects. The most serious of these is that, as a class, they are among the most potent teratogens known. Teratogens are compounds that cause severe birth defects during specific periods of fetal exposure. Other side effects include irritation of the tissues treated, which can be so severe that patients cannot tolerate treatment.

Various investigations have been undertaken to elucidate the structure-activity relationships governing the abilities of synthetic retinoids to induce the various pharmacological consequences of retinoic acid exposure. This has been a complicated task, however, since the assays available to investigators have been bioassays, carried out either in intact animals or in isolated tissues. Technical constraints have often dictated the use of different small animal species for different assays. Interpretation of results has been complicated by possible pharmacokinetic and metabolic effects and possible species differences in the receptors involved. Nevertheless, definite differences in the pharmacological effects of various synthetic retinoids have been observed.

Major insight into the molecular mechanism of retinoic acid signal transduction was gained in 1988. Prior to that time, several high abundance cellular retinoid binding proteins were incorrectly inferred to be the signal transducing receptors for retinoic acid. In 1988, a member of the steroid/thyroid hormone intracellular receptor superfamily (Evans, *Science,* 240:889-95 (1988)) was shown to transduce a retinoic acid signal (Giguere et al., *Nature,* 330:624-29 (1987); Petkovich *et al.*, *Nature,* 330: 444-50 (1987)). This unexpected finding related retinoic acid to other non-peptide hormones and elucidated the mechanism of retinoic acid effects in altering cell function. It is now known that retinoids regulate the activity of two distinct intracellular receptor subfamilies; the Retinoic Acid Receptors (RARs) and the Retinoid X Receptors (RXRs).

The first retinoic acid receptor identified, designated RAR-alpha, acts to modulate transcription of specific target genes in a manner which is ligand-dependent, as has been shown to be the case for many of the members of the steroid/thyroid hormone intracellular receptor superfamily. The endogenous low-molecular-weight ligand upon which the transcription-modulating activity of RAR-alpha depends is all-trans-retinoic acid. Retinoic acid receptor-mediated changes in gene expression result in characteristic alterations in cellular phenotype, with consequences in many tissues manifesting the biological response to retinoic acid. Two additional genes closely related to RAR-alpha were recently identified and were designated RAR-beta and RAR-gamma and are very highly related (Brand *et al., Nature,* 332:850-53 (1988); Ishikawa *et al., Mol. Endocrin.,* 4:837-44 (1990)). In the region of the retinoid receptors which can be shown to confer ligand binding, the primary amino acid sequences diverge by less than 15% among the three RAR subtypes or isoforms. All-*trans*-retinoic acid is a natural ligand for the retinoic acid receptors (RARs) and is capable of binding to these receptors with high affinity, resulting in the regulation of gene expression. The newly-discovered retinoid metabolite, 9-cis-retinoic acid, is also an activator of RARs.

A related but unexpected observation was made recently (Mangelsdorf *et al., Nature,* 345:224-29 (1990)), in which another member of the steroid/thyroid receptor superfamily was also shown to be responsive to retinoic acid. This new retinoid receptor subtype has been designated Retinoid X Receptor (RXR), because certain earlier data suggested that a derivative of all-*trans*-retinoic acid may be the endogenous ligand for RXR. Like the RARs, the RXRs are also known to have at least three subtypes or isoforms, namely RXR-alpha, RXR-beta, and RXR-gamma, with

corresponding unique patterns of expression (Manglesdorf et al., *Genes & Devel.,* 6:329-44 (1992)).

Although both the RARs and RXRs respond to all-trans-retinoic acid *in vivo,* the receptors differ in several important aspects. First, the RARs and RXRs are significantly divergent in primary structure (e.g., the ligand binding domains of RARα and RXRα have only 27% amino acid identity). These structural differences are reflected in the different relative degrees of responsiveness of RARs and RXRs to various vitamin A metabolites and synthetic retinoids. In addition, distinctly different patterns of tissue distribution are seen for RARs and RXRs. For example, in contrast to the RARs, which are not expressed at high levels in the visceral tissues, RXRa mRNA has been shown to be most abundant in the liver, kidney, lung, muscle and intestine. Finally, the RARs and RXRs have different target gene specificity. For example, response elements have recently been identified in the cellular retinal binding protein type II (CRBPII) and apolipoprotein AI genes which confer responsiveness to RXR, but not RAR. Furthermore, RAR has also been recently shown to repress RXR-mediated activation through the CRBPII RXR response element (Manglesdorf *et al., Cell,* 66:555-61 (1991)). These data indicate that two retinoic acid responsive pathways are not simply redundant, but instead manifest a complex interplay. Recently, Heyman *et al. (Cell,* 68:397-406 (1992)) and Levin *et al.* (*Nature,* 355:359-61 (1992)) independently demonstrated that 9-*cis*-retinoic acid is a natural endogenous ligand for the RXRs. 9-*cis*-retinoic acid was shown to bind and transactivate the RXRs, as well as the RARs, and therefore appears to act as a "bifunctional" ligand.

In view of the related, but clearly distinct, nature of these receptors, ligands which are more selective for the Retinoid X Receptor subfamily would be of great value for selectively controlling processes mediated by one or more of the RXR isoforms, and would provide the capacity for independent control of the physiologic processes mediated by the RXRs. Ligands which preferentially affect one or more but not all of the receptor isoforms also offer the possibility of increased therapeutic efficacy when used for medicinal applications.

EP-A 0260162 and GB-A 2197316 disclose bicyclic aromatic compounds and propose their use for treatment of dermatological conditions. However, these documents make no mention of selective activity of disclosed compounds on Retinoid X Receptors.

The entire disclosures of the publications and references referred to above and hereafter in this specification are incorporated herein by reference.

## SUMMARY OF THE INVENTION

The present invention now provides novel bicyclic aromatic compounds, which can be used to modulate processes mediated by one or more Retinoid X Receptors. More particularly, the invention relates to compounds which selectively or preferentially activate Retinoid X Receptors, in comparison to Retinoic Acid Receptors. These compounds selectively modulate processes mediated by Retinoid X Receptors. Accordingly, the invention also relates to methods for modulating processes selectively mediated by one or more Retinoid X Receptors, in comparison to Retinoic Acid Receptors, by use of the compounds of this invention. Examples of compounds used in and forming part of the invention include bicyclic benzyl, pyridinyl, thiophene, furanyl, and pyrrole derivatives. Pharmaceutical compositions containing the compounds disclosed are also within the scope of this invention. Also included are methods for identifying or purifying Retinoid X Receptors by use of the compounds of this invention.

## BRIEF DESCRIPTION OF THE FIGURES

The present invention may be better understood and its advantages appreciated by those skilled in the art by referring to the accompanying drawings wherein:

Figure 1 presents the standardized dose response profiles showing the transactivation of RAR and RXR isoforms by 3-methyl-TTNCB.

Figure 2 presents the standardized dose response profiles showing the transactivation of RAR and RXR isoforms by all-*trans*-retinoic acid.

Figure 3 presents the standardized dose response profiles showing the transactivation of RAR and RXR isoforms by 9-*cis*-retinoic acid.

Figure 4 presents the standardized dose response profiles showing the transactivation of RAR and RXR isoforms by 3-methyl-TTNEB.

Figure 5 presents the standardized dose response profiles showing the transactivation of RAR and RXR isoforms by 3-bromo-TTNEB.

Figure 6 presents the standardized dose response profiles showing the transactivation of RAR and RXR isoforms by 3-methyl-TTNCHBP.

Figure 7 presents the standardized dose response profiles showing the transactivation of RAR and RXR isoforms by 3-methyl-TTNEHBP.

Figure 8 presents the inhibition of transglutaminase activity by 9-*cis*-retinoid acid, all-*trans*-retinoic acid, and

3-methyl-TTNCB.

Figure 9 presents the Topical Dose Response, based on the test on Rhino mice, for 9-*cis*-retinoic acid, all-*trans*-retinoic acid, 3-methyl-TTNCB, 1, 25-dihydroxy Vitamin D.

Figure 10 presents the effect on rat HDL cholesterol of all-*trans*-retinoic acid, 9-*cis*-retinoic acid, 3-methyl-TTNCB, and 3-methyl-TTNEB.

Figure 11 presents the concentration-related effect of 3-methyl-TTNEB and TTNPB individually on incorporation of radiolabeled thymidine into DNA.

Figure 12 presents the concentration-related effect of a combination of 3-methyl-TTNEB and TTNPB on incorporation of radiolabeled thymidine into DNA.

DETAILED DESCRIPTION OF THE INVENTION

This invention discloses retinoid-like compounds or ligands which have selective activity for members of the subfamily of Retinoid X Receptors (RXRs), in comparison to members of the subfamily of Retinoic Acid Receptors (RARs). Examples of such compounds are bicyclic benzyl, pyridinyl, thiophene, furanyl, and pyrrole derivatives which can be represented by the formulae:

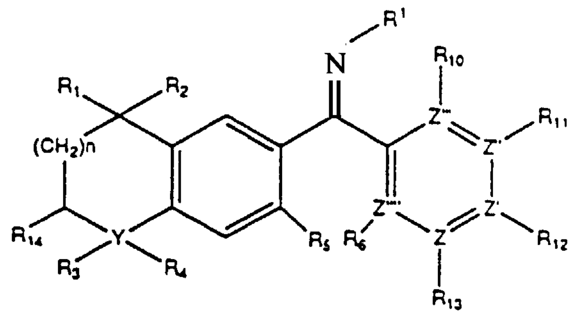

or

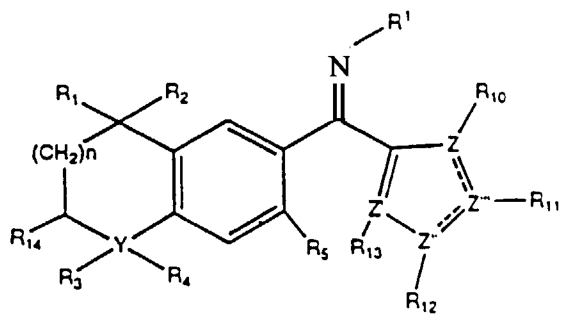

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;
$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' represents HO, NC, $(R_7R_8)N$, $R_{17}O$, $R_{17}$;
$R_5$ represents hydrogen, a lower alkyl having 1-4 carbons, halogen, nitro, $OR_7$, $SR_7$, $NR_7R_8$, or $(CF)_nCF_3$, with the proviso that if together $R_6$, $R_{10}$, $R_{11}$, $R_{12}$ and $R_{13}$ are all hydrogen, and Z, Z', Z'', Z''', and Z'''' are all carbon, or if R' represents OH, then $R_5$ is not hydrogen;
$R_6$, $R_{10}$, $R_{11}$, $R_{12}$, $R_{13}$ each independently represent hydrogen, a lower alkyl having 1-4 carbons, halogen, nitro,

$OR_7$, $SR_7$, $NR_7R_8$ or $(CF)_nCF_3$, and exist only if the Z, Z', Z", Z"', or Z"" from which it originates is C, or each independently represent hydrogen or a lower alkyl having 1-4 carbons if the Z, Z', Z", Z"', or Z"" from which it originates is N in a five-membered ring, and where one of $R_6$, $R_{10}$, $R_{11}$, $R_{12}$ or $R_{13}$ is X;

$R_7$ and $R_8$ each independently represents hydrogen or a lower alkyl having 1-6 carbons;

$R_9$ represents a lower alkyl having 1-4 carbons, phenyl, aromatic alkyl, or q-hydroxyphenyl, q-bromophenyl, q-chlorophenyl, q-florophenyl, or q-iodophenyl, where q=2-4;

$R_{14}$ represents hydrogen, a lower alkyl having 1-4 carbons, oxo, hydroxy, acyl having 1-4 carbons, halogen, thiol, or thioketone;

$R_{17}$ represents hydrogen, lower alkyl having 1-8 carbons, alkenyl (including halogen, acyl, $OR_7$ and $SR_7$ substituted alkenes), $R_9$, alkyl carboxylic acid (including halogen, acyl, OR, and SR, substituted alkyls), alkenyl carboxylic acid (including halogen, acyl, $OR_7$ and $SR_7$ substituted alkenes), alkyl amines (including halogen, acyl, $OR_7$ and $SR_7$ substituted alkyls), and alkenyl amines (including halogen, acryl, $OR_7$ and $SR_7$ substituted alkenes);

X is COOH, tetrazole, $PO_3H$, $SO_3H$, CHO, $CH_2OH$, $CONH_2$, COSH, $COOR_9$, $COSR_9$, $CONHR_9$, or COOW where W is a pharmaceutically acceptable salt, and where X can originate from any C or N on the ring; provided however that X cannot be COOH, CHO, $CH_2OH$, $CONH_2$, $COOR_9$ or COOW when X originates from a C in the 2 or 6 position on the ring;

Z, Z', Z", Z"' and Z"", each independently, represent C, S, O, N, or a pharmaceutically acceptable salt, but is not O or S if attached by a double bond to another such Z or if attached to another such Z which is O or S, and is not N if attached by a single bond to another such Z which is N;

n = 0-3; and

the dashed lines in the second structure shown depict optional double bonds.

As used in this disclosure, pharmaceutically acceptable salts include but are not limited to: hydrochloric, hydrobromic, hydroiodic, hydrofluoric, sulfuric, citric, maleic, acetic, lactic, nicotinic, succinic, oxalic, phosphoric, malonic, salicylic, phenylacetic, stearic, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydroxymethyl)aminomethane. Additional pharmaceutically acceptable salts are known to those of skill in the art.

Representative derivatives according to the present invention include the following:

4-[(3,5,5,8,8-pentamethyl-5,6,7,8-tetrahydro-2-naphthyl) carbonyl] benzoic acid oxime, designated Compound 112; and

4-[(3,5,5,8,8-pentamethyl-5,6,7,8-tetrahydro-2-naphthyl) carbonyl] benzoic acid methyloxime, designated Compound 115.

Representative structures for such compounds are as follows:

112

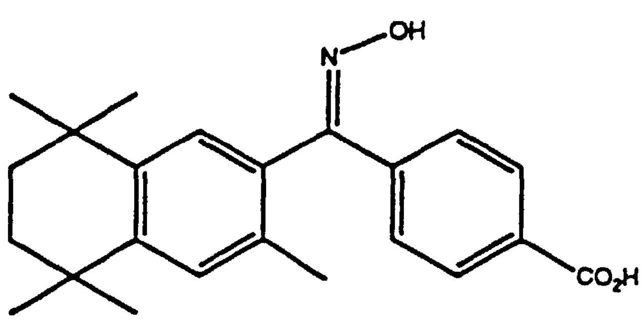

**4-[(3,5,5,8,8-pentamethyl-5,6,7,8-tetrahydro-2-naphthyl)carbonyl] benzoic acid oxime**

Et-112

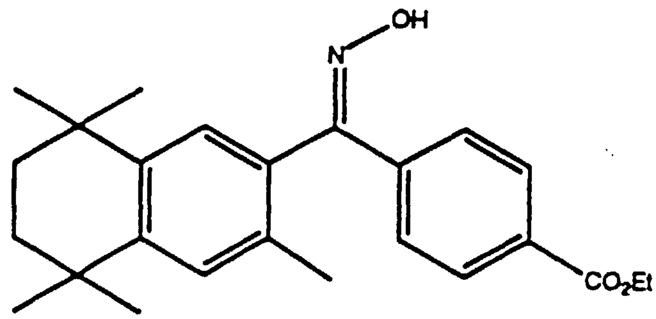

Ethyl-4-[(3,5,5,8,8-pentamethyl-5,6,7,8-tetrahydro-2-naphthyl)carbonyl] benzoate oxime

113

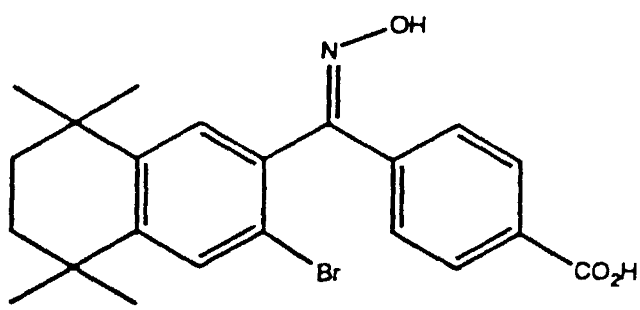

4-[(3-bromo-5,5,8,8-tetramethyl-5,6,7,8-tetrahydro-2-naphthyl) carbonyl] benzoic acid oxime

114

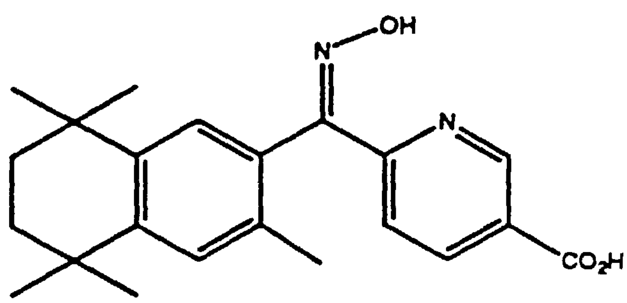

2-[(3,5,5,8,8-pentamethyl-5,6,7,8-tetrahydro-2-naphthyl)carbonyl] pyridine-5-carboxylic acid oxime

115

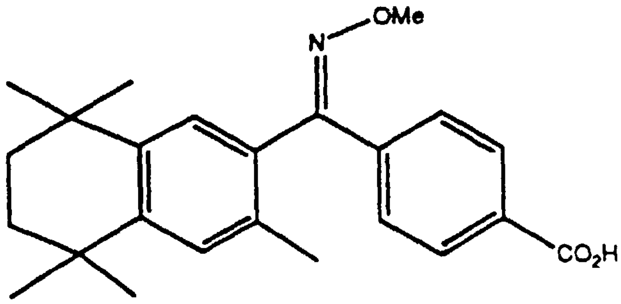

4-[(3,5,5,8,8-pentamethyl-5,6,7,8-tetrahydro-2-naphthyl) carbonyl] benzoic acid methyloxime

Et-115

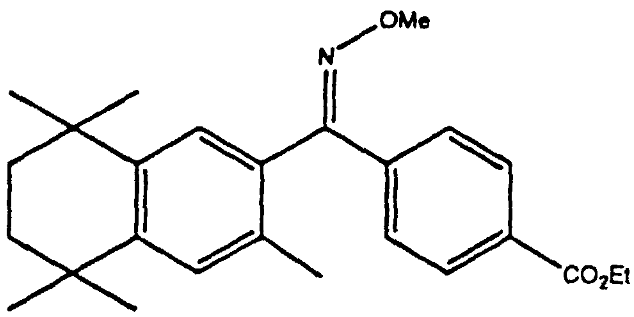

Ethyl-4-[(3,5,5,8,8-pentamethyl-5,6,7,8-tetrahydro-2-naphthyl)carbonyl] benzoate methyloxime

116

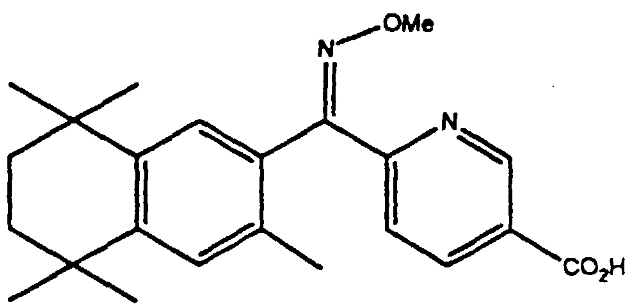

2-[(3,5,5,8,8-pentamethyl-5,6,7,8-tetrahydro-2-naphthyl) carbonyl] pyridine-5-carboxylic acid methyloxime

138 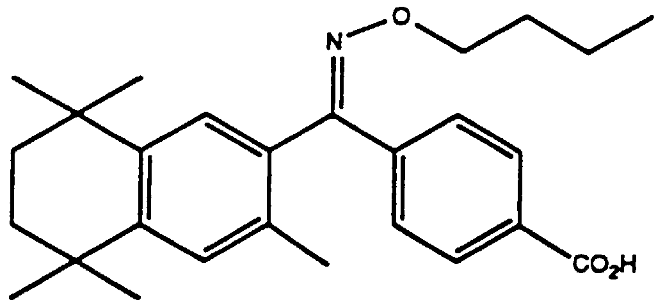

4-[(3,5,5,8,8-pentamethyl-5,6,7,8-tetrahydro-2-naphthyl) carbonyl] benzoic acid butyloxime

139 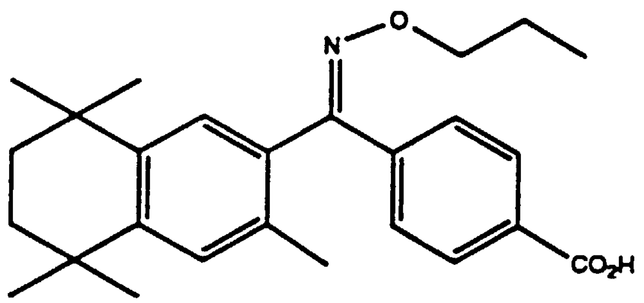

4-[(3,5,5,8,8-pentamethyl-5,6,7,8-tetrahydro-2-naphthyl) carbonyl] benzoic acid propyloxime

140 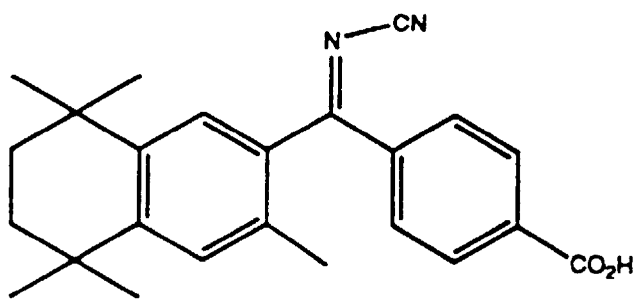

4-[(3,5,5,8,8-pentamethyl-5,6,7,8-tetrahydro-2-naphthyl) carbonyl] benzoic acid cyanoimine

141 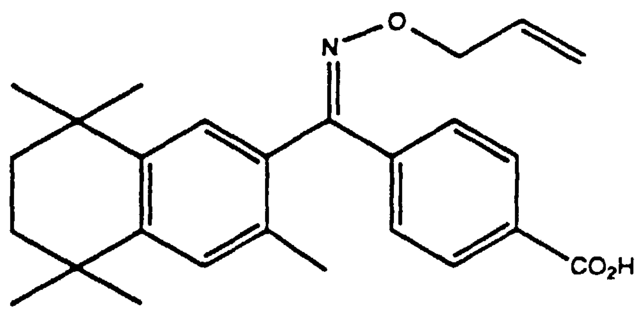

**4-[(3,5,5,8,8-pentamethyl-5,6,7,8-tetrahydro-2-naphthyl) carbonyl] benzoic acid allyloxime**

142 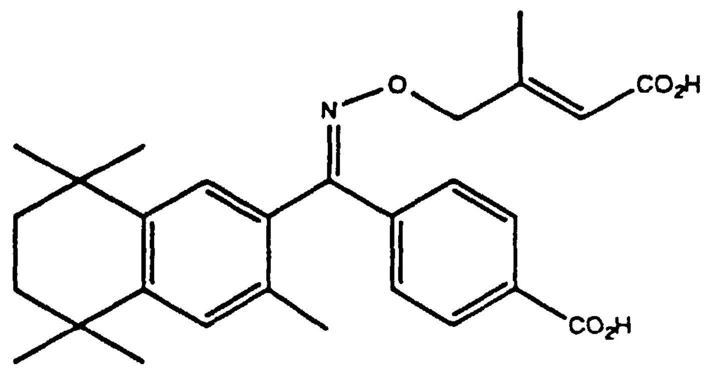

**4-[(3,5,5,8,8-pentamethyl-5,6,7,8-tetrahydro-2-naphthyl) carbonyl] benzoic acid 4-(3-methyl but-2-enoic acid)oxime**

143 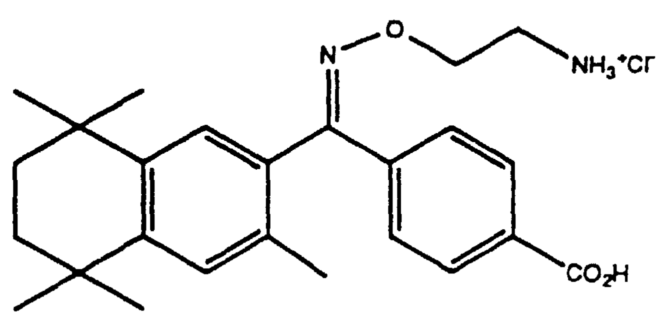

**4-[(3,5,5,8,8-pentamethyl-5,6,7,8-tetrahydro-2-naphthyl) carbonyl] benzoic acid 1-amino ethyloxime**

In addition, thiophene, furanyl, pyridine, pyrazine, pyrazole, pyridazine, thadiazole, and pyrrole groups function as isosteres for phenyl groups, and may be substituted for the phenyl group of the above bicyclic benzyl derivatives.

Representative derivatives of the present invention can be prepared according to the following illustrative synthetic schemes, in conjunction with the sections below relating to the "Synthesis of oximes and methyloximes", and

of alkyloximes and of cyanoimines:

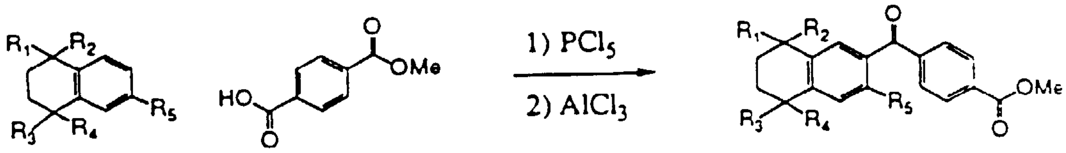


1 2 3

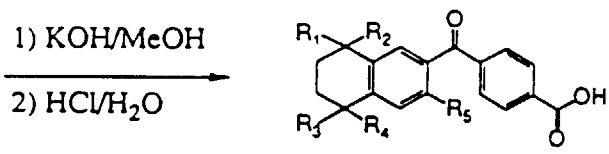


4

Compounds of structure 1 containing $R_5$ = lower alkyl are prepared in accordance with United States Patent No. 2,897,237. When $R_5$ = Halo, OH, amino, or thio, the products are prepared by standard Friedel-Crafts reaction conditions combining the appropriate substituted benzene with 2,5-dichloro-2,5-dimethyl hexane in the presence of aluminum trichloride.

Condensation of 1 with mono-methyl terephthalate 2 was carried out by addition of $PCl_5$ to 1 and 2 in $CH_2Cl_2$ followed by addition of $AlCl_3$ at room temperature.

The resulting methyl esters 3 are hydrolyzed to the carboxylic acid 4 by refluxing in aqueous KOH-MeOH followed by acidification.

4

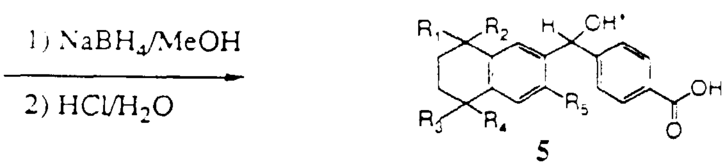


5

3

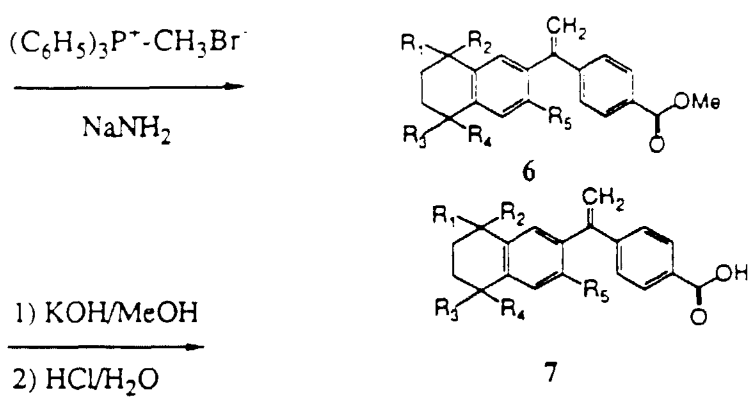


6

7

Treatment of ketone 4 with $NaBH_4$ afforded alcohol 5.

Treatment of the methyl ester 3 with methyltriphosphonium bromide-sodium amide in THF afforded the methano compound 6.

The carboxylic acid 7 was formed by adding KOH to methano compound 6 in MeOH, followed by acidification.

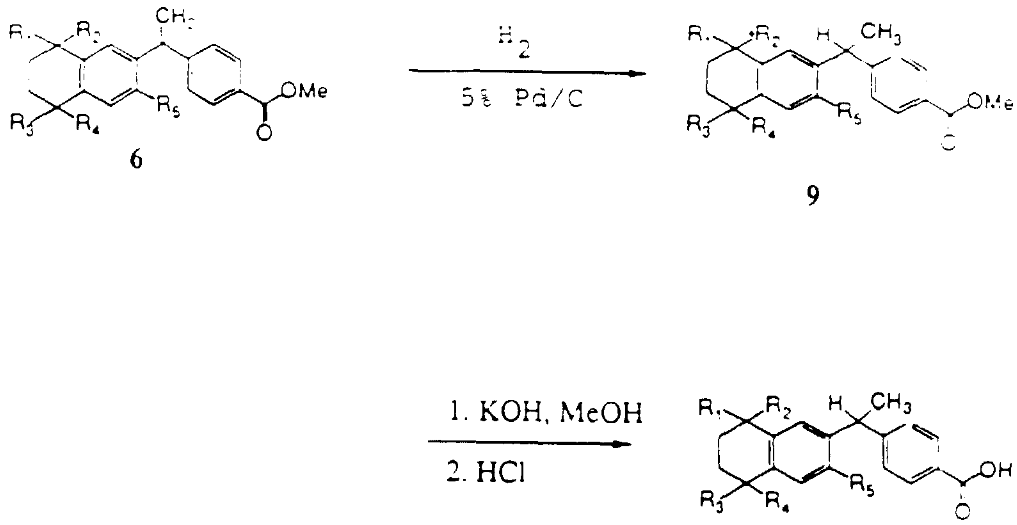


6

9

10

Treatment of the methyl ester 6 with hydrogen gas and 5% palladium over carbon in ethyl acetate yields the hydrogenated compound 9.

Treatment of compound 9 with aqueous KOH in refluxing MeOH, followed by acidification, yields the carboxylic acid compound 10.

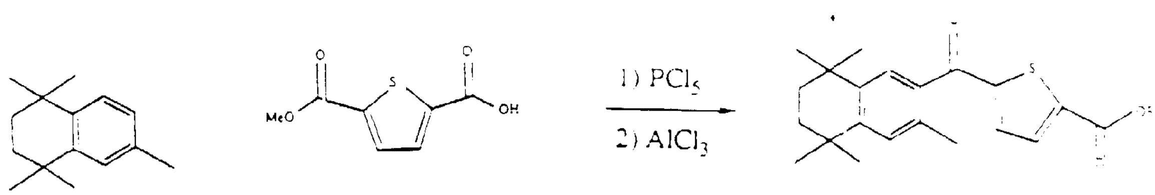


11

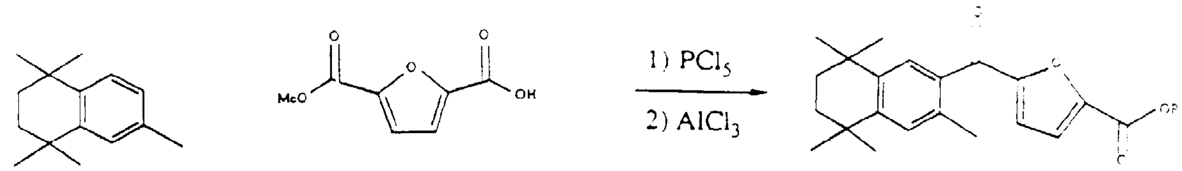


12

R = Me or H

Condensation of 1 with thiophene 2,5-mono methyl dicarboxylic acid or furanyl 2,5-mono methyl dicarboxylic acid was carried out by addition of $PCl_5$ in $CH_2Cl_2$ followed by addition of $AlCl_3$ at room temperature to give esters 11 and 12, which were hydrolyzed with KOH followed by acidification to the corresponding acids.

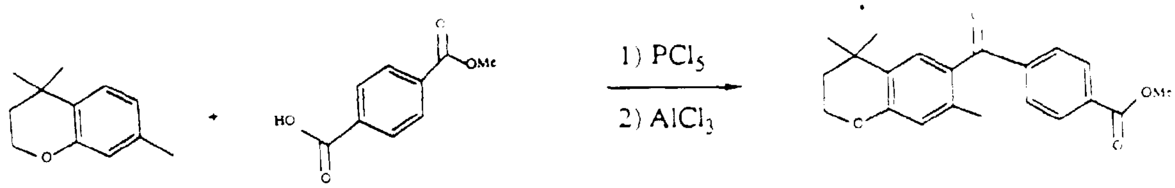


13

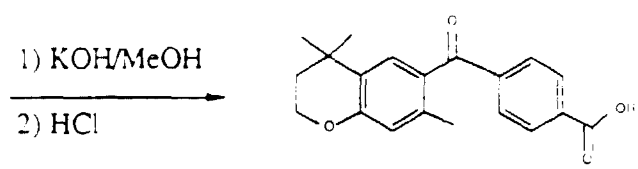


14

4,4-Dimethylchroman and 4,4-dimethyl-7-alkylchroman compounds of type 13 and 14 as well as 4,4-dimethylthiochroman, 4,4-dimethyl-7-alkylthiochroman, 4,4-dimethyl-1,2,3,4-tetrahydroquinoline, and 4,4-dimethyl-7-alkyl-1,2,3,4-tetrahydroquinoline analogs were synthesized by similar methods as compound 3, *i.e.*, Friedel-Crafts conditions combining the appropriate dimethylchroman, dimethylthiochroman or dimethyltetrahydroquinoline with mono-methyl terephthalate acid chloride in the presence of $AlCl_3$ or $SnCl_4$, followed by base hydrolysis and acidification to give the carboxylic acid. For the synthesis of the tetrahydroquinoline analogs, it was necessary to acylate the amine before Friedel-Crafts coupling with mono-methyl terephthalate acid chloride. For the synthesis of the appropriate dimethylchromans, dimethylthiochromans and tetrahydroquinolines, see U.S. Patent Nos. 5,053,523 and 5,023,341 and European Patent Publication No. 0284288.

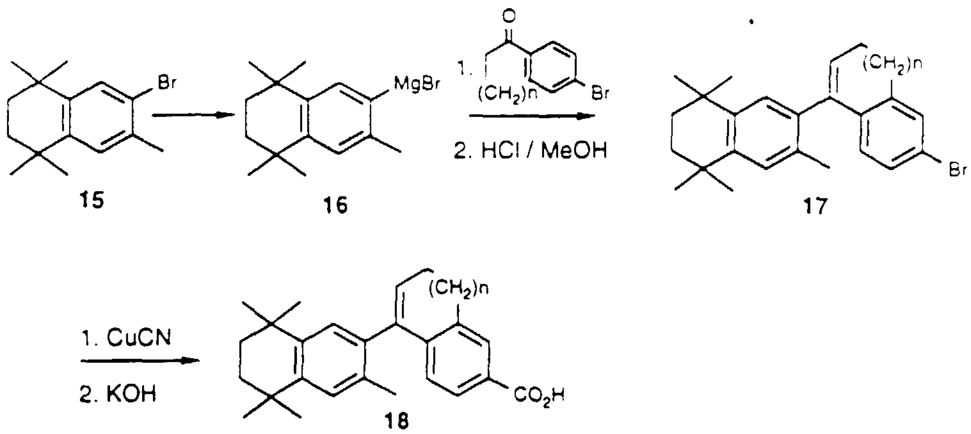


15 16 17 18

Compounds of the type 18 were synthesized by nucleophillic addition of the Grignard reagent 16 to bromotetralone, bromoindane, or other bicyclic ketone derivitive. Treatment of the resulting alcohol with methanolic HC1 gave the intermediate 17. Displacement of the bromine with CuCN in quinoline gave the nitrile which was then hydrolyzed to the acid 18 in refluxing KOH. Bromine compound 15 was synthesized from 2,5-dichloro-2,5-dimethylhexane and 2-bromotoluene with a catalytic amount of $AlCl_3$.

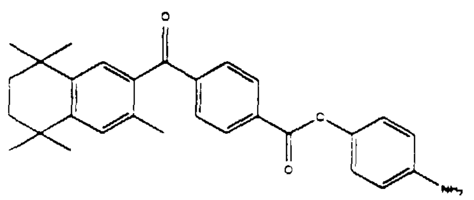

19

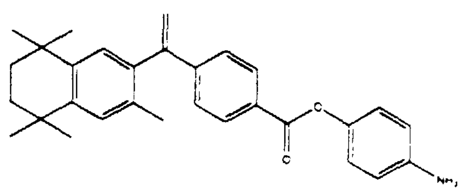

20

Treatment of compounds 3-methyl-TTNCB and 3-methyl-TTNEB with DCC, *p*-aminophenol, and DMAP resulted in the amino-esters 19 and 20.

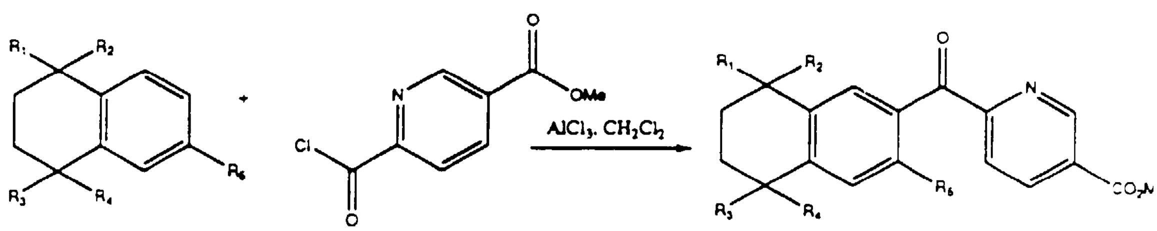


1 24 25

25

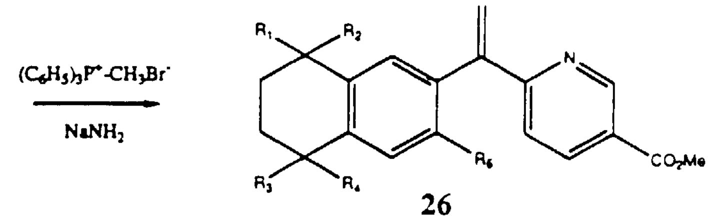


26

26

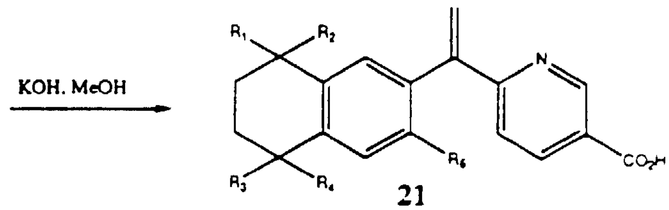


21

26

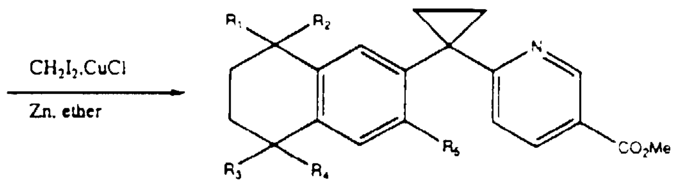


27

27

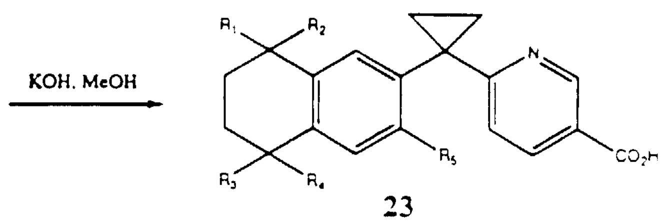


23

7

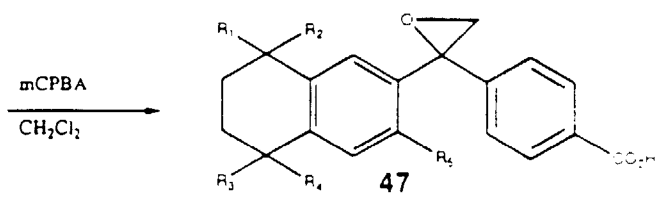


47

Representative pyridinal derivatives (compounds 21, 23, 26, and 27) may be prepared according to the illustrative synthetic schemes shown above. The synthesis of compound 21 is similar to that previously described for compound 7. Pentamethyl tetrahydronaphthalene 1, pyridinal acid chloride 24, and $AlCl_3$ are stirred in $CH_2Cl_2$ to give the ketone 25. Treatment of the ketone 25 with methyl triphosphonium bromide-sodium amide in THF afforded the ethenyl compound 26. Hydrolysis of 26 (KOH,MeOH) followed by acidification gave the acid 21. The cyclopropyl analog 23 was synthesized by treatment of the ethenyl compound 26 with $CH_2I_2$, zinc dust, CuCl in refluxing ether (Simmons-Smith reaction). Hydrolysis of the resulting cyclopropyl ester 27 was achieved with methanolic KOH followed by acidification to give compound 23. When $R_1$-$R_5$ are methyl, for example, compound 62 (TPNCP) is obtained, as shown in Example 33 below.

Other cyclopropyl derivatives such as TPNCB may be likewise prepared by the same method as described

for analog 23: olefin 6 is treated with the Simmons-Smith reagent described above, followed by hydrolysis with methanolic-KOH and acidification (HCl) to give the desired cyclopropyl derivative. Epoxy derivatives such as TPNEB may be synthesized by treatment of compound 7 with m-chloroperbenzoic acid at room temperature in $CH_2Cl_2$ for several hours.

Alternatively, pyridinal analogs, such as TPNEP, TPNEPC, and 3TTNEPE, may be prepared by the following synthetic route.

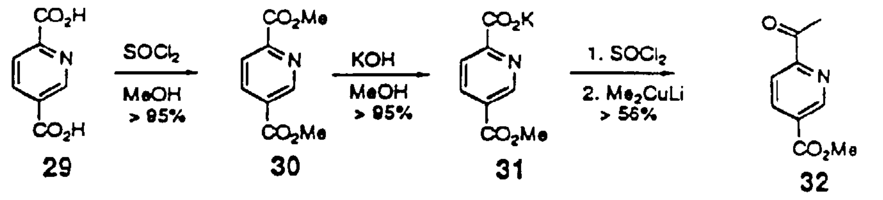

29 30 31 32

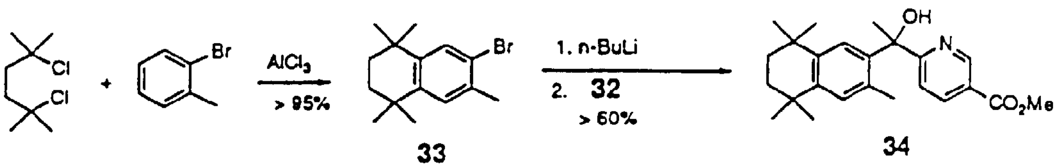

33 34

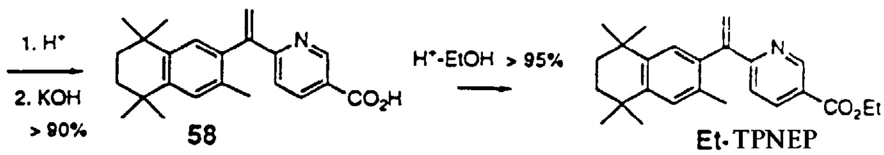

58 Et-TPNEP

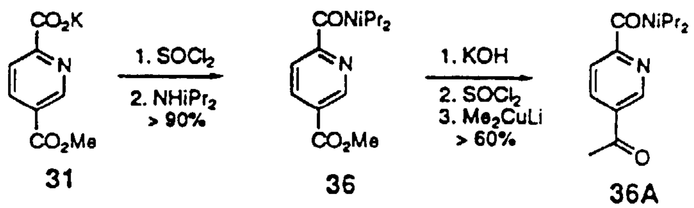

31 36 36A

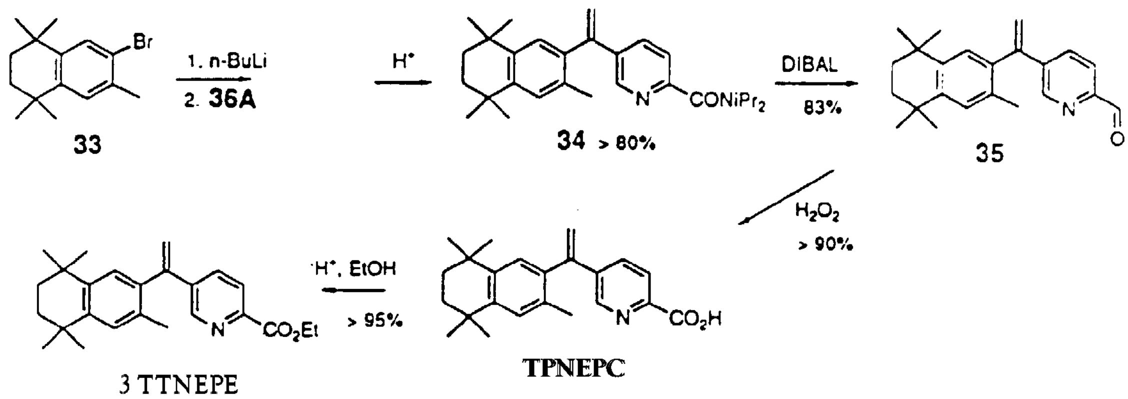


33 34 35

3 TTNEPE TPNEPC

# Synthesis of Oximes and Methyloximes

## Oxime from Carboxylic Acid:

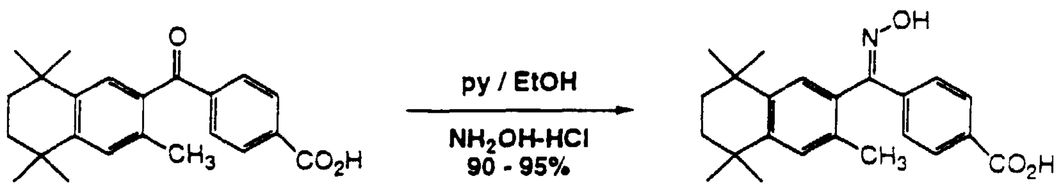


**3-methyl TTNCB** **112**

## Oximes from Esters:

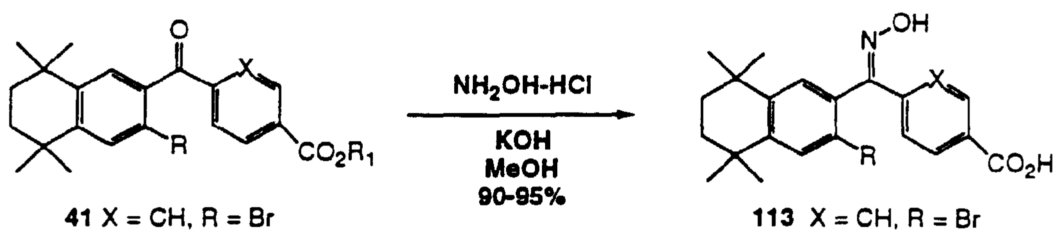


**41** X = CH, R = Br
**57** X = N, R = $CH_3$, $R_1$ = $CH_3$
$R_1$ = Me, Et, iPr

**113** X = CH, R = Br
**114** X = N, R = $CH_3$

## Methyloximes:

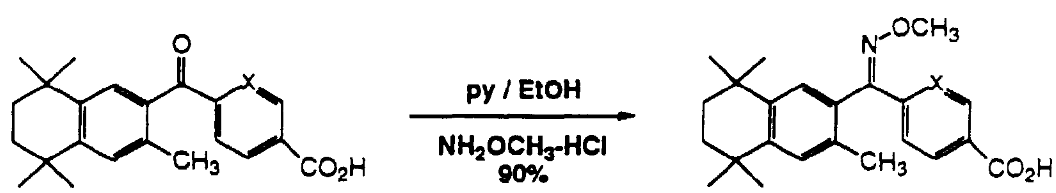


**3-methyl TTNCB** X = CH
**57** X = N

**115** X = CH
**116** X = N

## Synthesis of Alkyloximes

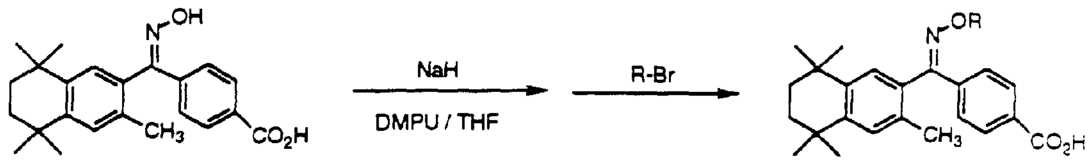


112

138 R = butyl
139 R = propyl
141 R = allyl
142 R = 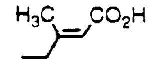

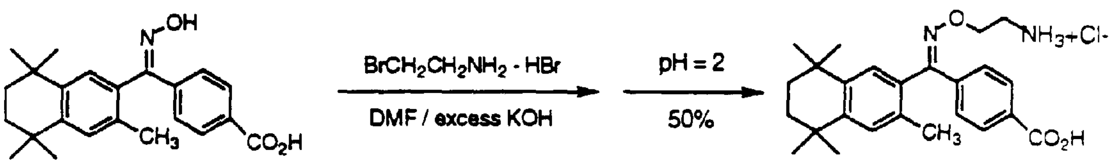


112

143

## Synthesis of Cyanoimine

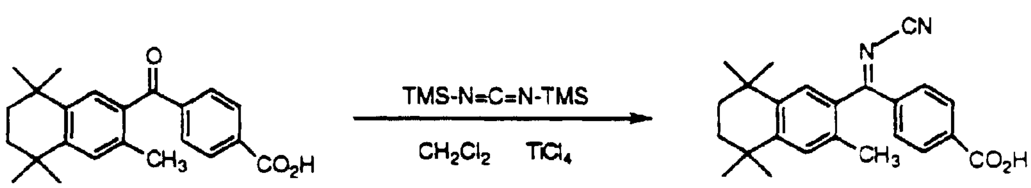


3-methyl TTNCB

140

Representative oxime derivatives (compounds 112, 113, and 114) may be prepared according to the illustrative synthetic schemes shown above. Synthesis of a representative methyloxime (compound 115) is also shown. A ketone such as 3-methyl-TTNCB is treated with hydroxylamine hydrochloride in pyridine and heated at reflux to give oxime 112. Alkyl oxime ethers are prepared from the corresponding ketone (such as 3-methyl-TTNCB) by treatment with methoxylamine hydrochloride in refluxing pyridine to give the methoxyomine 116. Also see Examples 44-49, below. Et-115 (and other ethyl esters such as Et-58, Et-62, Et-3-methyl-TTNEB) can be made by treatment of the respective carboxylic acids with oxalyl chloride to form the acid chloride, followed by treatment with EtOH and pyridine to give the ethyl ester. The oxime Et-112 can be made from Et-4-[(3,5,5,8,8-pentamethyl-5,6,7,8-tetrahydro-2-naphthyl)carbonyl] benzoate (Et-3-methyl TTNCB) by treatment with pyridine/EtOH and $NH_2OH$-CHI at reflux.

Substituted oximes (compounds 138-143) may also be prepared as shown above. These compounds were synthesized from the corresponding free oxime (112) by treatment of the oxime with NaH followed by alkylation with

the appropriate bromo alkyl group (R-Br).

Illustrative examples for the preparation of some of the compounds according to this invention are as follows:

Example 1

Preparation of compound 112, where $R_1$ $R_2$, $R_3$, $R_4$, $R_5$ are methyl; R' is HO ; X = COOH (oxime of 3-methyl-TTNCB):

3-methyl-TTNCB (4.41g, 12.6 mmol) in EtOH (10 mL) and pyridine (15.3 mL) was treated with hydroxylamine hydrochloride (4.38 g, 63 mmol), and the mixture was heated at reflux. After 6 h, the mixture was cooled to room temperature and the ethanol was removed *in vacuo*. The residue was taken-up in water and the aqueous layer was adjusted to pH = 4-5 with 1 M aqueous HCl. The aqueous solution was extracted 3 times with EtOAc; the organic layers were combined, and washed with water (2x) and brine. The organic solution was dried ($NaSO_4$), filtered, and concentrated to give a foamy white solid. Recrystallization ($CH_2Cl_2$/ether/ hexanes) gave a white solid, 4.05 g (88%). MP: 204-209°C(d); HRMS: 366.2060 (MH+);$^1$H-NMR ($CDCl_3$/d-4 MeOH) δ 1.22 (s, 6H, 2 ($CH_3$)), 1.32 (s, 6H, 2($CH_3$)), 1.69 (s, 4H, 2($CH_2$)), 2.11 (s, 3H, $CH_3$,), 6.99 (s, 1H, Ar-CH), 7.20 (s, 1H, Ar-CH), 7.53 (½ABq, 2H, J = 8.4 Hz, Δυ = 183.0 Hz, Ar-CH), 7.99 (½ ABq, 2H, J = 8.4 Hz, Δυ = 183.0 Hz-Ar-CH).

Example 2

Preparation of compound 113, where $R_1$, $R_2$, $R_3$, $R_4$ are methyl; $R_5$ is bromo; R' is HO ; X = COOH (oxime of 3-bromo-TTNCB, 41):

3-bromo-TTNCB methyl ester (399 mg, 0.93 mmol) in MeOH (2 mL) was treated with hydroxylamine hydrochloride (97 mg. 1.4 mmol) and KOH (156 mg, 2.8 mmol), and the mixture was heated at reflux for 3 h. The reaction was worked-up in a manner identical to that described for 112 to give a white solid 330 mg (83%). MP: 240-244°C (d); $^1$H-NMR ($CDCl_3$) δ 1.26 (s, 6H, 2 ($CH_3$)), 1.33 (s, 6H, 2 ($CH_3$)), 1.72 (s, 4H, 2($CH_2$)), 7.30 (s, 1H, Ar-CH), 7.54 (s, 1H, Ar-CH), 7.92 (½ABq, 2H, J = 8.3 Hz Δυ = 113.5 Hz, Ar-CH), 8.20 (½ABq, 2H, J = 8.3 Hz Δυ = 113.5 Hz, Ar-CH).

Example 3

Preparation of compoupd 114, where $R_1$, $R_2$, $R_3$, $R_4$, $R_5$ are methyl; R' is HO ; X = COOH; Z = N; Z', Z'', Z''' = CH (oxime of 57):

The compound was prepared in a manner similar to that described for 113, except compound 57, rather than 3-methyl-TTNCB, was the starting ketone. Flash chromatography on $SiO_2$, (hexanes:EtOAC;$CH_2Cl_2$:isopropanol = 10: 5:1:1) of the crude product, gave a sticky, white solid, 83 mg (86%). MP: 167-172°C(d); HRMS: 367.2025($MH^+$); FTIR (neat) 3600-3230 (br), 2962, 2928, 2664, 1697, 1593, 1296, 1273, 1122, 1028, 964 $cm^{-1}$ $^1$H-NMR ($CDCl_3$), δ 1.23 (s, 6H, 2 ($CH_3$)), 1.32 (s, 6H, 2($CH_3$)), 169 (s, 4H, 2 ($CH_2$)), 2.14 (s, 3H, $CH_3$), 7.05 (s, IH, Ar-CH), 7.22 (s, 1H, Ar-CH), 7.49 (½ABq, 2H, J = 28.0 Hz, Ar-CH), 8.25 (½ABq, 2H, J= 8.0 Hz, Ar-CH); 9.21 (s, IH, Ar-CH).

Example 4

Preparation of compound 115, where $R_1$, $R_2$, $R_3$, $R_4$, $R_5$ are methyl; R' is $CH_3O$ ; X = COOH (methoxyoxime of 3-methyl-TTNCB):

3-methyl-TTNCB (560 mg, 1.60 mmol) in EtOH (2 mL) and pyridine (1.3 mL) was treated with methoxylamine hydrochloride (402 g, 4.81 mmol), and the mixture was heated at reflux. After 6 h, the mixture was cooled to room temperature and the ethanol was removed *in vacuo*. The residue was taken-up in water and the aqueous layer was adjusted to pH = 4-5 with 1 M aqueous HCl. The aqueous solution was extracted 3 times with EtOAc, the organic layers were combined, and washed with water (2x) and brine. The organic solution was dried ($NaSO_4$), filtered', concentrated, and crystallized ($CH_2Cl_2$/hexanes) to give a white solid, 564 mg (93%). MP:228-228.5°C; LRMS: 380 ($MH^+$); $^1$H-NMR ($CDCl_3$) δ 1.22 (s, 6H, 2 ($CH_3$)), 1.31 (s, 6H, 2 ($CH_3$)), 1.69 (s, 4H, 2($CH_2$)), 2.08 (s, 3H, $CH_3$), 4.01 (s, 3H, $OCH_3$), 6.95 (s, 1H, Ar-CH), 7.18 (s, 1H, Ar-CH), 7.57 (½ABq, 2H, J = 8.4 Hz, Δυ = 188.3 Hz, Ar-CH), 8.04 (½ABq, 2H, J = 8.4 Hz, Δυ = 188.3 Hz, Ar-CH).

Example 5

Preparation of compound 116, where R1, R2, R3, R4, R5 are methyl; R' is $CH_3O$ ; X=COOH, Z=N; Z',Z", Z"' = CH (methyloxime of 57):

3-methyl-TTNCB methyl ester (151 mg, 0.41 mmol) in EtOH (1 mL) was treated with methoxylamine hydrochloride (52 mg, 0.62 mmol) and pyridine (70 μL, 0.82 mmol), and the mixture was heated at reflux for 5 h. The reaction was worked-up in a manner identical to that described for 115 to give a solid (169 mg). The crude product was hydrolyzed in excess KOH/MeOH at ambient temperature for 24 h. The methanol was removed *in vacuo.* The residue was taken-up in water and the aqueous layer was adjusted to pH = 4-5 with 1 M aqueous HCl. The aqueous solution was extracted 3 times with EtOAc; the organic layers were combined, and washed with water (2x) and brine. The organic solution was dried ($NaSO_4$), filtered, concentrated, and crystallized ($Et_2O$/hexanes) to give a white solid, 96 mg (61%). MP: 260-263°C; $^1$H-NMR ($CDCl_3$) δ 1.22 (s, 6H, 2($CH_3$)), 1.30 (s, 6H, 2($CH_3$)), 1.68 (s, 4H, 2($CH_2$)), 2.10 (s, 3H, $CH_3$), 4.08 (s, 3H, $OCH_3$), 6.99 (s, 1H, Ar-CH), 7.18 (s, 1H, Ar-CH), 7.63 (d, 1H, Py-CH, J = 8.0 Hz), 8.31 (d, 1H, Py-CH, J = 8.0 Hz), 9.31 (s, 1H, Py-CH).

Example 6

Preparation of compound 138, where R1, R2, R3, R4, R5 are methyl; R' is n-BuO ; X = COOH (n-butyloxime of 3-methyl-TTNCB):

A solution of the oxime of 3-methyl-TTNCB (compound 112, 121 mg, 0.33 mmol) in THF (0.3 mL) and DMPU (0.3 mL) was added at 0°C to a suspension of NaH (24 mg, 1.0 mmol) in THF (1.0 mL). The suspension was allowed to warm to room temperature with stirring over 30 minutes, then a solution of n-butyl bromide (136 mg, 110 μL, 1.0 mmol) in THF (1.0 mL) was added. The solution was allowed to warm to room temperature and stirred. for 15 hr. Aqueous, saturated $NH_4Cl$ (3.0 mL) was added and the aqueous layer was adjusted to pH = 4-5 with 1 M aqueous HCl. The aqueous solution was extracted 3 times with EtOAc; the organic layers were combined, and washed with water (2x) and brine. The organic solution was dried ($MgSO_4$), filtered, concentrated, and crystallized (ether/hexanes) to give a white solid, 82 mg (58%). MP: 195-198°C; $^1$H-NMR ($CDCl_3$) δ0.94 (t, 3H, $CH_3$, J = 7.4 Hz), 1.22 (s, 6H, 2 ($CH_3$)), 1.31 (s, 6H, 2($CH_3$)), 1.69 (s, 4H, 2($CH_2$)), 1.72 (mult, 2H, $CH_2$, J = 7.3 Hz), 2.05 (s, 3H, $CH_3$), 4.16 (t, 3H, $OCH_2$, J = 6.7 Hz), 6.97 (s, 1H, Ar-CH), 7.16 (s, 1H, Ar-CH), 7.57 (d, 2H, Ar-$CH_2$, J = 8.4 Hz), 8.04 (d, 2H, Ar-$CH_2$, J = 8.4 Hz).

Example 7

Preparation of compound 139, where R1, R2, R3, R4, R5 are methyl; R' is n-ProO ; X = COOH (n-propyloxime of 3-methyl-TTNCB):

A solution of the oxime of 3-methyl-TTNCB (compound number 112, 121 mg, 0.33 mmol) in THF (0.3 mL) and DMPU (0.3 mL) was added at 0°C to a suspension of NaH (24 mg, 1.0 mmol) in THF (1.0 mL). The suspension was allowed to warm to room temperature with stirring over 30 minutes, then a solution of n-propyl bromide (122 mg, 90 μL, 1.0 mmol) was added. The solution was allowed to warm to room temperature and stirred for 15 hr. Aqueous, saturated $NH_4Cl$ (5.0 mL) was added and the aqueous layer was adjusted to pH = 4-5 with 1 aqueous HCl. The aqueous solution was extracted 3 times with EtOAc; the organic layers were combined, and washed with water (2x) and brine. The organic solution was dried ($MgSO_4$), filtered, concentrated, and crystallized (ether/hexanes) to give a white solid, 99 mg (73%). MP: 178-180°C; $^1$H-NMR ($CDCl_3$) δ 0.93 (t, 3H, $CH_3$, J = 7.4 Hz), 1.26 (s, 6H, 2($CH_3$)), 1.35 (s, 6H, 2 ($CH_3$)), 1.51 (mult, 2H, $CH_2$, J = 7.4 Hz), 1.73 (mult, 6H, 2($CH_2$)/$CH_2$), 2.09 (s, 3H, $CH_3$), 4.24 (t, 3H, $OCH_2$J = 7.4 Hz), 7.00 (s, 1H, Ar-CH), 7.19 (s, 1H, Ar-CH), 7.60 (d, 2H, Ar-$CH_2$, J = 8.5 Hz), 8.07 (d, 2H, Ar-$CH_2$, J = 8.5 Hz).

Example 8

Preparation of compound 140, where R1, R2, R3, R4, R5 are methyl; R' is CN ; X = COOH (cyanoimine of 3-methyl-TTNCB);

A solution of 3-methyl-TTNCB (350 mg, 1.0 mmol) in methylene chloride (1.5 mL) was treated with bis(trimethylsilyl)carbodiimide (186 mg, 230 μL, 1.0 mmol) at room temperature. The solution was cooled to 0°C, and treated with a 1M solution of $TiCl_4$ in methylene chloride (2 eq., 2 mL, 2 mmol). The resulting dark red solution was heated at reflux for 8 hr. An additional equivalent of $TiCl_4$ (1 mL of 1M methylene chloride solution) was added and the reflux

was continued for 3 hours, and TLC analysis indicated complete conversion to one product. The solution was cooled to room temperature, then poured into ice cold aqueous $NaHSO_4$. The mixture was diluted with ethyl acetate and filtered through a pad of celite. The aqueous layer was washed twice with ethyl acetate and the organic layers were combined. The organic solution was washed twice with water and once with saturated aqueous NaCl solution, dried ($Na_2$ $SO_4$), filtered, and concentrated. The residue was crystallized from solution with methylene chloride/hexanes/benzene to give a pale yellow solid, 207 mg (55%). MP: 217-219°C; IR (KBr pellet) 3500-2600 br, 2961, 2924, 2666, 2210, 1693, 1582, 1560, 1427, 1408, 1314, $cm^{-1}$; $^1$H-NMR ($CDCl_3$) δ 1.26 (s, 6H, 2($CH_3$)), 1.32 (s, 6H, 2($CH_3$)), 1.72 (s, 4H, 2($CH_2$)), 2.11 (s, 3H, $CH_3$), 7.14 (s, 1H, Ar-CH), 7.24 (s, 1H, Ar-CH), 7.90 (d, 2H, Ar-CH, J = 8.1 Hz), 8.17 (d, 2H, Ar-CH, J = 8.1 Hz); $^{13}$C-NMR ($CDCl_3$) δ 191.3, 170.5, 148.6, 143.1, 140.4, 133.9, 132.7, 131.8, 130.4, 129.2, 125.9, 34.8, 34.7, 34.4, 34.1, 31.7, 31.6, 19.5; FAB MS $C_{24}H_{26}N_2O_2$: 375 ($MH^+$).

Example 9

Preparation of compound 141, where R1, R2, R3, R4, R5 are methyl; R' is allylO ; X = COOH (allyloxime of 3-methyl-TTNCB):

A solution of the oxime of 3-methyl-TTNCB (compound number 112, 100 mg, 0.27 mmol) in THF (0.3 mL) and DMPU (0.3 mL) was added at 0°C to a suspension of NaH (20 mg, 0.82 mmol) in THF (1.0 mL) . The suspension was allowed to warm to room temperature with stirring over 30 minutes, then a solution of allyl bromide (71 µL, 0.82 mmol) was added. The solution was stirred at room temperature for an additional 12 hr. Aqueous, saturated $NH_4Cl$ (5.0 mL) was added and the aqueous layer was adjusted to pH = 4-5 with 1 M aqueous HCl. The aqueous solution was extracted 3 times with EtOAc; the organic layers were combined, and washed with water (2x) and brine. The organic solution was dried ($MgSO_4$), filtered, concentrated, and crystallized (ether/hexanes) to give a white solid, 87 mg (78%). $^1$H-NMR ($CDCl_3$) δ 1.21 (s, 6H, 2($CH_3$)),1.31 (s, 6H, 2($CH_3$)), 1.69 (s, 4H, 2($CH_2$)), 2.06 (s, 3H, $CH_3$), 4.71 (d, 2H, $OCH_2$, J = 5.6 Hz), 5.19 (dd, 1H, =$CH_{trans}$, J=10.4, 1.5 Hz), 5.27 (dd, 1H, = $CH_{cis}$, J = 17.2, 1.5 Hz), 6.02 (mult, 1H, =CH), 6.97 (s, 1H, Ar-CH), 7.16 (s, 1H, Ar-CH), 7.57 (d, 2H, Ar-CH, J = 8.2 Hz), 8.03 (d, 2H, Ar-CH, J = 8.2 Hz).

Example 10:

Preparation of compound 142, where R1, R2, R3, R4, R5 are methyl; R' is HOOC-C=C($CH_3$)$CH_2$-O ; X = COOH (4-(3-methyl-butenyl-1-carboxy)oxime of 3-methyl-TTNCB):

A solution of the oxime of 3-methyl-TTNCB (compound number 112, 202 mg, 0.55 mmol) in THF (0.5 mL) and DMPU (0.5 mL) was added at 0°C to a suspension of NaH (38 mg, 1.7 mmol) in THF (1.7 mL). The suspension was allowed to warm to room temperature with stirring over 30 minutes, then a solution of ethyl 4-bromo-3-methyl-but-2-ene carboxylate (343 mg, 1.7 mmol) was added. The solution was allowed to warm to room temperature and stirred for 12 hr. Aqueous, saturated $NH_4Cl$ (5.0 mL) was added and the aqueous layer was adjusted to pH = 4.5 with 1 M aqueous HCl. The aqueous solution was extracted 3 times with EtOAc; the organic layers were combined, and washed with water (2x) and brine. The organic solution was dried ($MgSO_4$), filtered, and concentrated to give a yellow oil. Radial chromatography (1 mm $SiO_2$ plate, 9 : 1 = hexane; EtOAc with gradual addition of 2% isopropanol) gave a yellow oil. The ester was hydrolyzed in excess KOH/MeOH at room temperature for 24 hr. The methanol was removed *in vacuo.* The residue was taken-up in water and the aqueous layer was adjusted to pH = 4-5 with 1 M aqueous HCl. The aqueous solution was extracted 3 times with EtOAc; the organic layers were combined, and washed with water (2x) and brine. The organic solution was dried ($NaSO_4$), filtered, concentrated, and crystallized ($Et_2O$/hexanes) to give a white solid, 110 mg (43%). $^1$H-NMR ($CDCl_3$) δ 1.25 (s, 6H, 2($CH_3$)), 1.31 (s, 6H, 2($CH_3$)), 1.70 (s, 4H, 2($CH_2$)), 2.06 (s, 3H, Ar-$CH_3$), 2.14 (s, 3H, $CH_3$), 4.71 (s, 2H, $OCH_2$), 5.85 (s, 1H, =CH), 7.01 (s, 1H, Ar-CH), 7.16 (s, 1H, Ar-CH), 7.55 (d, 2H, Ar-$CH_2$, J = 8.2 Hz), 8.02 (d, 2H, Ar-$CH_2$, J = 8.2 Hz).

Example 11

Preparation of compound 143, where R1, R2, R3, R4, R5 are methyl; R' is Cl-$NH_3$+$CH_2CH_2$-O ; X = COOH (2-aminoethyloxime of 3-methyl-TTNCB):

To a solution of the oxime of 3-methyl-TTNCB (compound number 112, 108 mg, 0.30 mmol) and 2-bromoethylamine hydrobromide (182 mg, 0.89 mmol) in DMF (1 mL) was added excess powered KOH at 0°C. The yellow solution was allowed to warm to room temperature and stirred for 48 hr. Aqueous, saturated $NH_4Cl$ (5.0 mL) was added and the aqueous layer was adjusted to pH = 2 with 1 M aqueous HCl. The aqueous solution was extracted 3 times with EtOAc; the organic layers were combined, and washed with water (2x) and brine. The organic solution was dried

($MgSO_4$), filtered, and concentrated, and recrystallized ($Et_2O$/hexanes) to give a white solid, 64 mg (50%). IR (KBr pellet) 3600-3200 broad, 3420, 2922, 2855, 1695, 1591, 1456, 1364, 1231, 1017 $cm^{-1}$; $^1H$-NMR ($CDCl_3/CD_3OD$) $\delta$ 1.21 (s, 6H, 2($CH_3$)), 1.29 (s, 6H, 2($CH_3$)), 1.70 (s, 4H, 2 ($CH_2$)), 2.06 (s, 3H, Ar-$CH_3$), 2.14 (s, 3H, $CH_3$), 3.29 (m, 2H, $CH_2$), 4.37 (m, 2H, $CH_2$), 7.00 (s, 1H, Ar-CH), 7.24 (s, 1H, Ar-CH), 7.50 (d, 2H, Ar-$CH_2$, J = 8.0 Hz), 7.94 (d, 2H, Ar-$CH_2$, J = 8.0 Hz).

Evaluation of Retinoid Receptor Subtype Selectivity

Representative synthetic retinoid compounds of the current invention were analyzed and found to exhibit subtype selectivity for retinoid receptors, and to be capable of modulating processes selectively mediated by retinoid X receptors, as discussed more fully below.

As employed herein, the phrase "processes selectively mediated by retinoid X receptors" refers to biological, physiological, endocrinological, and other bodily processes which are mediated by receptors or receptor combinations which are responsive to retinoid X receptor selective processes, *e.g.*, compounds which selectively activate one and/or multiple members of the RXR subfamily. Modulation includes activation or enhancement of such processes as well as inhibition or repression, and can be accomplished *in vitro* or *in vivo. In vivo* modulation can be carried out in a wide range of subjects, such as, for example, humans, rodents, sheep, pigs, cows, and the like. It is well accepted that modulation of such processes has direct relevance to use in treating disease states.

The receptors which are responsive to retinoid X receptor selective ligands include: retinoid X receptor-alpha, retinoid X receptor-beta, retinoid X receptor-gamma, and splicing variants encoded by the genes for such receptors, as well as various combinations thereof *(i.e.,* homodimers, homotrimers, heterodimers, heterotrimers, and the like). Also included are combinations of retinoid X receptors with other members of the steroid/thyroid superfamily of receptors with which the retinoid X receptors may interact by forming heterodimers, heterotrimers, and the higher heteromultimers. For example, the retinoic acid receptor-alpha, -beta, or -gamma isoforms form a heterodimer with any of the retinoid X receptor isoforms, (*i.e.,* alpha, beta, or gamma, including any combination of the different receptor isoforms), and the various retinoid X receptors form a heterodimer with thyroid receptor and form a heterodimer with vitamin D receptor. Members of the retinoid X receptor subfamily form a heterodimer with certain "orphan receptors" including PPAR (Issemann and Green, *Nature*, 347:645-49 (1990)); HNF4 (Sladek *et al., Genes & Development* 4:2353-65 (1990)); the COUP family of receptors (*e.g.*, Miyajima *etal., Nucleic Acids Research* 16:11057-74 (1988), and Wang *et al., Nature,* 340:163-66 (1989)); COUP-like receptors and COUP homologs, such as those described by Mlodzik *et al. (Cell,* 60:211-24 (1990)) and Ladias *et al. (Science,* 251:561-65 (1991)); the ultraspiracle receptor (*e.g.*, Oro *et al., Nature,* 347:298-301 (1990)); and the like.

As employed herein, the phrase "members of the steroid/thyroid superfamily of receptors" (also known as "nuclear receptors" or "intracellular receptors") refers to hormone binding proteins that operate as ligand-dependent transcription factors. Furthermore, this classification includes identified members of the steroid/thyroid superfamily of receptors for which specific ligands have not yet been identified (referred to hereinafter as "orphan receptors"). All members of the intracellular receptor superfamily have the intrinsic ability to bind to specific DNA sequences. Following binding, the transcriptional activity of a target gene (*i.e.*, a gene associated with the specific DNA sequence) is modulated as a function of the ligand bound to the receptor. Also, see Heyman *et al., Cell*, 68:397-406 (1992), and copending U.S. Serial No. 809,980, filed December 18, 1991, whose entire disclosures are incorporated herein by reference.

The modulation of gene expression by the ligand retinoic acid and its receptors can be examined in a reconstituted system in cell culture. Such a system was used to evaluate the synthetic retinoid compounds of this invention for their interaction with the retinoid receptor subtypes RAR$\alpha$, RAR$\beta$, RAR$\gamma$, RXR$\alpha$, RXR$\beta$, and RXR$\gamma$.

The system for reconstituting ligand-dependent transcriptional control, which was developed by Evans *et al., Science,* 240:889-95 (1988), has been termed a "co-transfection" or *"cis-trans*" assay. This assay is described in further detail in U.S. Patent Nos. 4,981,784 and 5,071,773, which are incorporated herein by reference. Also see Heyman *et al., Cell,* 68:397-406 (1992). The co-transfection assay provides a mechanism to evaluate the ability of a compound to modulate the transcription response initiated by an intracellular receptor. The co-transfection assay is a functional, rapid assay that monitors hormone or ligand activity, is a good predictor of an *in vivo* system, and can be used to quantitate the pharmacological potency and utility of such ligands in treating disease states. Berger *et al., J. Steroid Biochem. Molec. Biol.,* 41:733-38 (1992).

Briefly, the co-transfection assay involves the introduction of two plasmids by transient transfection into a retinoid receptor-negative mammalian cell background. The first plasmid contains a retinoid receptor cDNA and directs constitutive expression of the encoded receptor. The second plasmid contains a cDNA that encodes for a readily quantifiable protein, e.g., firefly luciferase or chloramphenicol acetyl transferase (CAT), under control of a promoter containing a retinoid acid response element, which confers retinoid dependence on the transcription of the reporter. In this co-transfection assay, all retinoid receptors respond to all-*trans*-retinoic acid in a similar fashion. This assay can be used to accurately measure efficacy and potency of retinoic acid and synthetic retinoids as ligands that interact with

the individual retinoid receptor subtypes.

Accordingly, synthetic retinoid compounds of the current invention were evaluated for their interaction with retinoid receptor subtypes using the co-transfection assay in which CV-1 cells were co-transfected with one of the retinoid receptor subtypes, a reporter construct, and an internal control to allow normalization of the response for transfection efficiency. The following example is illustrative.

Example 12

Retinoids: All-trans-retinoic acid (RA) and *13-cis*-retinoic acid (13-*cis*-RA) were obtained from Sigma. 9-*cis*-retinoic acid (9-*cis*-RA) was synthesized as described in Heyman *et al., Cell,* 68:397-406 (1992). Retinoid purity was established as greater than 99% by reverse phase high-performance liquid chromatography. Retinoids were dissolved in dimethylsulfoxide for use in the transcriptional activation assays.

Plasmids: The receptor expression vectors used in the co-transfection assay have been described previously (pRShRAR-α: Giguere *et al.* (1987); pRShRAR-β and pRShRAR-γ: Ishikawa *et al.* (1990); pRShRXR-α: Mangelsdorf *et al.,* (1990); pRSmRXR-β and pRSmRXR-γ: Mangelsdorf *et al., Genes* & *Devel.,* 6:329-44 (1992)). A basal reporter plasmid Δ-MTV-LUC (Hollenberg and Evans, *Cell,* 55:899-906 (1988)) containing two copies of the TRE-palindromic response element 5'-TCAGGTCATGACCTGA-3' (Umesono *et al., Nature,* 336:262-65 (1988)) was used in transfections for the RARs, and CRBPIIFKLUC, which contains an RXRE (retinoid X receptor response element (Mangelsdorf *et al., Cell,* 66:555-61 (1991)), was used in transfections for the RXRs.

Co-transfection Assay In CV-1 Cells: A monkey kidney cell line, CV-1, was used in the *cis-trans* assay. Cells were transfected with two plasmids. The trans-vector allowed efficient production of the retinoid receptor in these cells, which do not normally express this receptor protein. The *cis*-vector contains an easily assayable gene product, in this case the firefly luciferase, coupled to a retinoid-responsive promoter, *i.e.*, an RARE or RXRE. Addition of retinoic acid or an appropriate synthetic retinoid results in the formation of a retinoid-RAR or-RXR complex that activates the expression of luciferase gene, causing light to be emitted from cell extracts. The level of luciferase activity is directly proportional to the effectiveness of the retinoid-receptor complex in activating gene expression. This sensitive and reproducible co-transfection approach permits the identification of retinoids that interact with the different receptor isoforms.

Cells were cultured in DMEM supplemented with 10% charcoal resin-stripped fetal bovine serum, and experiments were conducted in 96-well plates. The plasmids were transiently transfected by the calcium phosphate method (Umesono and Evans, *Cell,* 57:1139-46 (1989) and Berger *et al., J. Steroid Biochem. Molec. Biol.,* 41:733-38 (1992)) by using 10 ng of a pRS (Rous sarcoma virus promoter) receptor-expression plasmid vector, 50 ng of the reporter luciferase (LUC) plasmid, 50 ng of pRSβ-GAL(β-galactosidase) as an internal control, and 90 ng of carrier plasmid, pGEM. Cells were transfected for 6 h and then washed to remove the precipitate. The cells were then incubated for 36 h with or without retinoid. After the transfection, all subsequent steps were performed on a Beckman Biomek Automated Workstation. Cell extracts were prepared, then assayed for luciferase and β-galactosidase activities, as described by Berger *et al.* (1992). All determinations were performed in triplicate in two independent experiments and were normalized for transfection efficiency by using β-galactosidase as the internal control. Retinoid activity was normalized relative to that of all-*trans*-retinoic acid and is expressed as potency (EC50), which is the concentration of retinoid required to produce 50% of the maximal observed response, and efficacy (%), which is the maximal response observed relative to that of all-*trans*-retinoic acid at $10^{-5}$M. The data obtained is the average of at least four independent experiments. Efficacy values less than 5% are not statistically different than the 0% background. Compounds with an efficacy of less than 20% at concentrations of $10^{-5}$ M are considered to be inactive. At higher concentrations of compound, such as $10^{-4}$ M, these compounds are generally toxic to cells and thus the maximal efficacy at $10^{-5}$ M is reported in the tables and figures contained herein.

The synthetic retinoid compound 3-methyl-TTNCB, as described above, was evaluated for its ability to regulate gene expression mediated by retinoid receptors. As shown in Figure 1, this compound is capable of activating members of the RXR subfamily, *i.e.,* RXRα, RXRβ, and RXRγ, but clearly has no significant activity for members of the RAR subfamily, *i.e.,* RARα, RARβ, and RARγ. Assays using all-*trans*-retinoic acid (Figure 2) and 9-*cis*-retinoic acid (Figure 3) were run for reference, and demonstrate that these retinoic acid isomers activate members of both the RAR and RXR subfamilies.

Potency and efficacy were calculated for 3-methyl-TTNCB compound (not covered by the present claims), as summarized in the following table. For reference, the data for 9-*cis*-retinoic acid are also included. Later, we we provide data showing potency and efficiency for oximes of the present invention.

TABLE 1

| | Potency (nM) | Efficacy |
|---|---|---|
| 3-Methyl-TTNCB | | |
| RXRα | 330 | 130% |
| RXRβ | 200 | 52% |
| RXRγ | 260 | 82% |
| RARα | >10,000 | <2% |
| RARβ | >10,000 | <4% |
| RARγ | >10,000 | <4% |
| 9-cis-retinoic acid | | |
| RXRα | 150 | 140% |
| RXRβ | 100 | 140% |
| RXRγ | 110 | 140% |
| RARα | 160 | 100% |
| RARβ | 5 | 82% |
| RARγ | 47 | 120% |

As shown by the data in Table 1, 3-methyl-TTNCB readily and at low concentrations activates RXRs. Further, 3-methyl-TTNCB is more potent an activator of RXRs than RARs, and preferentially activates RXRs in comparison to RARs, in that much higher concentrations of the compound are required to activate the RARs. In contrast, 9-*cis*-retinoic acid does not preferentially activate the RXRs, as also shown in Table 1. Rather, 9-*cis*-retinoic acid activates the RARβ and RARγ isoforms at lower concentrations and more readily than the RXRβ and RXRγ isoforms, and has substantially the same, within the accuracy of the measurement, activity for the RARα isoform in comparison to the RXRα isoform.

An extract reported to contain 9-*cis*-retinoic acid has previously been reported as at least 10-fold more potent in inducing RXRα than RARα (Heyman *et al., Cell,* 68:397,399 (January 24, 1992)). Presently available data indicate that 9-*cis*-retinoic acid does not preferentially activate RXRs in comparison to RARs, as shown and discussed above. The compounds of this invention preferentially activate RXRs in comparison to RARs, and are preferably at least three times more potent as activators of RXRs than RARs, and more preferably at least five times more potent as activators of RXRs than RARs.

Potency and efficacy have also been calculated for other comparative compounds, namely 3-methyl-TTNEB, 3-bromo-TTNEB, 3-methyl-TTNCHBP, 3-methyl-TTNEHBP, TPNEP, and TPNCP compounds, as summarized below in Table 2.

TABLE 2

| | Potency (nM) | Efficacy |
|---|---|---|
| 3-Methyl-TTNEB | | |
| RXRα | 40 | 83% |
| RXRβ | 21 | 102% |
| RXRγ | 34 | 80% |
| RARα | >10,000 | 6% |
| RARβ | >10,000 | 17% |
| RARγ | >10,000 | 19% |
| 3-Bromo-TTNEB | | |
| RXRα | 64 | 88% |
| RXRβ | 54 | 49% |

TABLE 2 (continued)

| Potency (nM) | | Efficacy |
|---|---|---|
| 3-Bromo-TTNEB | | |
| RXRγ | 52 | 71% |
| RARα | >10,000 | 3% |
| RARβ | >10,000 | 18% |
| RARγ | >10,000 | 15% |
| 3-Methyl-TTNCHBP | | |
| RXRα | 1100 | 113% |
| RXRβ | 1100 | 155% |
| RXRγ | 300 | 128% |
| RARα | >10,000 | <2% |
| RARβ | >10,000 | 7% |
| RARγ | >10,000 | 17% |
| 3-Methyl-TTNEHBP (63) | | |
| RXRα | 140 | 125% |
| RXRβ | 71 | 121% |
| RXRγ | 48 | 163% |
| RARα | >10,000 | <2% |
| RARβ | 1,900 | 25% |
| RARγ | >10,000 | 10% |
| TPNEP (58) | | |
| RXRα | 5 | 75% |
| RXRβ | 5 | 138% |
| RXRγ | 6 | 100% |
| RARα | >10,000 | <2% |
| RARβ | >10,000 | <2% |
| RARγ | 1,500 | 24% |
| TPNCP (62) | | |
| RXRα | 4 | 63% |
| RXRβ | 4 | 93% |
| RXRγ | 3 | 49% |
| RARα | >10,000 | <2% |
| RARβ | >10,000 | <2% |
| RARγ | >10,000 | <2% |

As shown by the data in Table 2, 3-methyl-TTNEB, 3-bromo-TTNEB, 3-methyl-TTNCHBP, 3-methyl-TTNEHBP, TPNEP, and TPNCP each readily and preferentially activate the RXRs, and are more potent as activators of RXRs than of RARs. The diminished activity of these compounds for the RARs in comparison to the RXRs is also shown for some of these compounds in Figures 4-7.

The potency and efficacy of the oxime derivative compounds 112 and 115 have also been calculated, as

summarized below in Table 3.

TABLE 3

| | Potency (nM) | Efficacy |
|---|---|---|
| Oxime Cd. 112 | | |
| RXRα | 15 | 66% |
| RXRβ | 8 | 51% |
| RXRγ | 12 | 62% |
| RARα | >10,000 | 3% |
| RARβ | >10,000 | 3% |
| RARγ | >10,000 | 3% |
| Oxime Cd. 115 | | |
| RXRα | 5 | 61% |
| RXRβ | 5 | 71% |
| RXRγ | 5 | 70% |
| RARα | >10,000 | 4% |
| RARβ | >10,000 | 2% |
| RARγ | >10,000 | 3% |

As shown, oxime compounds 112 and 115 preferentially activate RXRs in comparison to RARs.

The selective activity of the compounds of this invention for Retinoid X Receptors is not exhibited by other known compounds. For example, compounds such as those described in U.S. Patent No. 4,833,240 (Maignan et al.) appear structurally similar to the compounds of this invention, but lack a functional group (such as methyl, ethyl, isopropyl, bromo, chloro, etc.) at the 3-position. Such compounds have little or no potency and lack any selectivity for RXRs.

For example, a representative compound of U.S. Patent No. 4,833,240 (Maignan) is shown below.

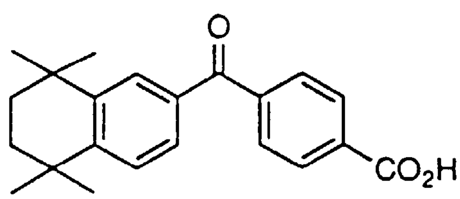

Maignon Ex. II

The potency and efficacy of the Maignon compound are summarized below:

| | Potency (nM) | Efficacy |
|---|---|---|
| Maignon Ex. II | | |
| RXRα | 3,000 | 82% |
| RXRβ | 3,000 | 44% |
| RXRγ | 3,000 | 64% |
| RARα | >10,000 | 11% |

(continued)

| | Potency (nM) | Efficacy |
|---|---|---|
| Maignon Ex. II | | |
| RARβ | 1,900 | 58% |
| RARγ | 2,000 | 56% |

As shown, the Maignon compound is virtually inactive and shows no selectivity for RXRs. In contrast, the compounds of this invention which have a substituant at the 3-position, are potent activators of RXRs and exhibit the unexpected RXR selectivity shown in Table 3 and discussed above.

It can be expected that synthetic retinoid ligands, such as those exemplified in Table 3 which preferentially affect some but not all of the retinoic acid receptor isoforms, can, in pharmacological preparations, provide pharmaceuticals with higher therapeutic indices and a better side effect profile than currently used retinoids. For example, the compounds of the present invention have been observed to be less irritating to the skin than previously known retinoids.

The retinoid compounds of this invention are useful for the treatment of certain dermatological conditions such as keratinization disorders, *i.e.*, differentiation/proliferation. A standard assay to determine the activity of these compounds is the measurement of the enzymatic activity for transglutaminase; this is a measure of the antiproliferative action of retinoids. Retinoids have been shown to inhibit the pathway of differentiation, which is indicated by a decrease in several biochemical markers that are associated with the expression of squamous cell phenotype, such as transglutaminase. (Yuspa *et al., Cancer Research,* 43 :5707-12 (1983)). As can be seen from Figure 8, the 3-methyl-TTNCB compound is capable of inhibiting transglutaminase activity and inhibits 50% of the enzyme activity at 1 x $10^{-7}$ M.

The retinoid compounds of this invention have been demonstrated in *in vitro* tests to block (or antagonize) AP-1 activity, a set of oncogenes which drive cellular proliferation. Many proliferative disorders are the results of oncogenes/oncogene activation, and therefore a compound which blocks the AP-1 oncogene pathway can be used to treat diseases associated with proliferative disorders including cancers, inflammatory diseases, psoriasis, etc. For example, the compound 3-methyl-TTNEB was evaluated using the co-transfection assay in which HeLa cells were co-transfected with a plasmid expressing RXRα under the control of a constitutive promoter, and a plasmid which expresses the reporter enzyme luciferase under the control of a conditional promoter (collagenase) containing an AP-1 responsive element. *E.g.,* Angel et *al., Mol. Cell. Biol.,* 7:2256 (1987); Lafyatis *et al., Mol. Endocrinol.,* 4:973 (1990). Following AP-1 activation, the assay results showed antagonism of AP-1 activity by the 3-methyl-TTNEB compound via RXRα in a dose-dependent manner. Other compounds of this invention have shown similar antagonism of AP-1 activity. These results demonstrate that RXR-selective compounds such as 3-methyl-TTNEB can be used as anti-proliferatives to limit cell growth and treat diseases associated with hyperproliferation.

The compounds of this invention also exhibit good comedolytic activity in the test on Rhino mice described by Kligman *et al.(J. of Inves. Derm.,* 73:354-58 (1979)) and Mezick et *al. (J. of Inves. Derm.,* 83:110-13 (1984)). The test on Rhino mice has been a model for screening comedolytic agents. The activity of the 3-methyl-TTNCB retinoid compound, as well as 9-*cis* and all-*trans* retinoic acid is shown in Figure 9. A 0.1% solution of 3-methyl-TTNCB is capable of inhibiting the utriculi diameter by approximately 50%. It has also been observed that 3-methyl-TTNCB is less irritating to the skin of Rhino mice than *9-cis-* or all-*trans*-retinoic acid.

The co-transfection assay allows examination of the ability of a compound to modulate gene expression in a retinoid receptor dependent fashion. To examine the ability of the compounds of this invention to directly interact with the receptors, we have examined the ligand binding properties of all six retinoid receptors. The receptors were expressed employing a baculovirus expression system in which we have demonstrated that RXRα binds 9-*cis*-retinoic acid with high affinity (Heyman et *al.,* Cell, 68:397 (1992)). The binding parameters of receptors expressed in baculovirus systems and mammaliam systems are essentially identical.

The synthetic retinoids of the current invention have also been tested using radioligand displacement assays. By testing the abilities of various synthetic retinoids to compete with the radiolabeled retinoic acid for binding to various receptor isoforms, the ability of the compounds to directly interact with the receptor can be examined, and the relative dissociation constant for the receptor itself can be determined. This is an important supplementary analysis to the co-transfection assay since it can detect different properties/determinants of retinoid activity than are measured in the co-transfection assay. These determinants/differences in the two assay systems may include (1) activating or inactivating metabolic alterations of the test compounds, (2) binding to serum proteins which could alter the free concentration or other properties of the test compound, (3) differences in cell permeation among test compounds, (4) intrinsic differences in the affinity of the test compounds for the receptor proteins, *i.e.*, in $K_d$, can be directly measured, and (5) conformational changes produced in the receptor after binding of the test compound, reflected in the effects on reporter gene expression; (*i.e.*, a functional measurement of receptor activation).

The 3-methyl-TTNCB compound is capable of displacing $^3$H-9-*cis*-retinoic acid bound to the RXRs, but is not

capable of displacing radiolabeled ligand that is bound to the RARs. This indicates that the 3-methyl-TTNCB compound preferentially binds RXRs in comparison to RARs, a property which would be expected of a ligand selective for the RXRs.

The $K_d$ values were determined by application of the Cleng-Prusoff equation. These values were based on a determination of the $IC_{50}$ value as determined graphically from a log-logit plot of the data.

Binding data were obtained for various compounds using the method discussed in Wecksler & Norman, Anal. *Biochem.* 92:314-23 (1979). Results are shown below in Table 4.

TABLE 4

| 3-Methyl-TTNCB | Binding ($Kd_{50}$ nM) |
|---|---|
| RXRα | 350 |
| RXRβ | 230 |
| RXRγ | 365 |
| RARα | >10,000 |
| RARβ | >10,000 |
| RARγ | >10,000 |
| 3-Methyl-TTNEB | |
| RxRα | 41 |
| RXRβ | 20 |
| RXRγ | 22 |
| RARα | 5,500 |
| RARβ | 5,400 |
| RARγ | 3,200 |
| TPNEP | |
| RXRα | 22 |
| RXRβ | 21 |
| RXRγ | 39 |
| RARα | 7,800 |
| RARβ | 4,900 |
| RARγ | 6,000 |
| TPNCP | |
| RXRα | 3 |
| RXRβ | 3 |
| RXRγ | 3 |
| RARα | >10,000 |
| RARβ | >10,000 |
| RARγ | >10,000 |
| Oxime Cd. 112 | |
| RXRα | 6 |
| RXRβ | 5 |
| RXRγ | 5 |

TABLE 4 (continued)

| 3-Methyl-TTNCB | Binding ($Kd_{50}$ nM) |
|---|---|
| Oxime Cd. 112 | |
| RARα | 8,700 |
| RARβ | >10,000 |
| RARγ | >10,000 |

The compounds in Table 4 were shown to readily and preferentially activate RXRs, and to be more potent as activators of RXRs than of RARs using the co-transfection assay, as discussed above. The binding results in Table 4 show these compounds to also preferentially bind RXRs versus RARs. Taken together the ligand binding properties of these compounds and their ability to selectively modulate members of the RXR subfamily demonstrate the identification of a class of compounds with the unique biological properties. The binding properties and especially the transcriptional activation assays are a good predictor of the pharmacological activity of a compound (Berger *et al.* (1992)).

It has been recognized that the co-transfection assay provides a functional assessment of the ligand being tested as either an agonist or antagonist of the specific genetic process sought to be affected, and is a predictor of *in vivo* pharmacology (Berger et al. (1992)). Ligands which do not significantly react with other intracellular receptors, as determined by the co-transfection assay, can be expected to result in fewer pharmacological side effects. Because the co-transfection assay is run in living cells, the evaluation of a ligand provides an early indicator of the potential toxicity of the candidate at concentrations where a therapeutic benefit would be expected.

Processes capable of being modulated by retinoid receptors, in accordance with the present invention, include *in vitro* cellular differentiation, the regulation of morphogenetic processes including limb morphogenesis, regulation of cellular retinol binding protein (CRBP), and the like. As readily recognized by those of skill in the art, the availability of ligands for the retinoid X receptor makes it possible, for the first time, to elucidate the processes controlled by members of the retinoid X receptor subfamily. In addition, it allows development of assays for the identification of antagonists for these receptors.

The processes capable of being modulated by retinoid receptors, in accordance with the present invention, further include the *in vivo* modulation of lipid metabolism; *in vivo* modulation of skin related processes (e.g., acne, psoriasis, aging, wrinkling, and the like); *in vivo* modulation of programmed cell death (apoptosis); *in vivo* modulation of malignant cell development, such as occurs, for example, in acute promyelocytic leukemia, mammary cancer, prostate cancer, lung cancer, cancers of the aerodigestive pathway, skin cancer, bladder cancer, and sarcomas; *in vivo* modulation of premalignant lesions, such as occurs with oral leukoplakia and the like; *in vivo* modulation of auto-immune diseases such as rheumatoic arthritis; *in vivo* modulation of fatty acid metabolism; and the like. Such applications can be expected to allow the modulation of various biological processes with reduced occurrence of undesirable side effects such as teratogenic effects, skin irritation, mucosal dryness, lipid disturbances, and the like. *In vivo* applications can be employed with a wide range of subjects, such as, for example, humans, rodents, sheep, pigs, cows, and the like.

For example, regarding the *in vivo* modulation of lipid metabolism referred to above, apolipoprotein A-1 ("apoA1") is a major protein component of plasma high density lipoprotein (HDL) cholesterol. The circulating level of HDL in humans has been shown to be inversely correlated to the risk of atherosclerotic cardiovascular disease (ASCVD), the leading cause of morbidity and mortality in the United States, with a 3-4% increase in ASCVD for every 1% decrease in HDL cholesterol. Gordon *et al., New Engl. J. Med.,* 321:1311 (1989). While there are currently no good therapeutic regimens that increase HDL cholesterol, it can be expected that regulating synthesis of apoAI can be utilized to affect plasma concentrations of HDL cholesterol and to decrease the risk of ASCVD. Rubin *et al., Nature,* 353:265 (1991).

It has been established that regulation of transcription of apoAI is controlled by members of the intracellular receptor superfamily, and further that the apoAI gene transcription start site "A" is a highly selective retinoic acid-responsive element that responds to retinoid X receptors. Rottman *et al., Mol. Cell. Biol.,* 11:3814-20 (1991). Because RXRs can form heterodimers with trans-repressers such as ARP-1 and COUP-TF and transactivators such as HNF-4, and an RXR response element resides in the apoAI promoter, retinoids or ligands which selectively activate members of the RXR family of retinoic acid receptors may regulate apoAI transcription. We have demonstrated in *in vivo* studies that ligands of this invention which have selective activity for RXRs can be used to modulate apoA1/HDL cholesterol and to significantly raise plasma HDL levels, as demonstrated in the following study.

Male Sprague-Dawley rats (160-200 gram) were obtained from Harlan. Animals were fed standard laboratory diets (Harlan/Teklad) and kept in an environmentally controlled animal house with a light period lasting from 6 a.m. to 6 p.m. Animals were treated with drugs prepared as suspensions in olive oil.

To verify that RXR activation can increase plasma apoAI/HDL cholesterol, an initial study was carried out that included dosing rats for 4 days with an RAR-selective compound, all-*trans* retinoic acid, the non-selective RAR/RXR

agonist, 9-*cis*-retinoic acid, and either of two RXR-selective agents, 3-methyl-TTNCB or 3-methyl-TTNEB. Each drug was administered at a dose of 100 mg/kg, i.p. Positive control groups received olive oil as a vehicle. Twenty-four hours after the last treatment, rats were sacrificed by $CO_2$ inhalation, blood was collected from the inferior vena cava into a tube containing 0.1 ml of 0.15% EDTA and centrifuged at 1500 x g for 20 min. at 4°C, Plasma was separated and stored at 4°C for evaluation of plasma total cholesterol and high density lipoprotein cholesterol (HDL-cholesterol).

Plasma total cholesterol was measured enzymatically utilizing Boeringer Mannheim Diagnostics High Performance Cholesterol Methods with an ABBOTT VP Bichromatic Analyzer. HDL cholesterol was measured after preparation of the HDL-containing fraction by heparin-manganese precipitation of plasma. HDL-cholesterol in this fraction was estimated as mentioned earlier. All HDL separations were checked for contamination by other lipoproteins with agarose gel electrophoresis.

The results of this study are shown in Figure 10. As shown, rats receiving the RXR-selective compounds exhibited substantial and statistically significant increases in HDL levels, particularly when receiving 3-methyl-TTNEB.

Because the RXR-selective ligand 3-methyl-TTNEB was the most efficacious, additional 4 day experiments were conducted with this agent at doses of 0.3, 1, 3, 6, 10, 30, 100, or 300 mg/kg i.p. in 1.0 ml olive oil or 1, 3, 10, 30, 100, 300 mg/kg p.o. in 1.0 ml olive oil for 4 days. An additional 30 day p.o. study was conducted with 10, 30, or 100 mg/kg 3-methyl-TTNEB to determine whether tolerance would develop to its pharmacological actions. For the rats receiving 3-methyl-TTNEB in various doses for four days, it was observed that 3-methyl-TTNEB increased plasma concentrations of HDL-cholesterol in a dose-dependent manner with significant increases being made at the lowest dose, 0.3 mg/kg i.p. At its optimally efficacious dose, 3-methyl-TTNEB increased plasma HDL-cholesterol concentrations from 58 mg/dl to 95 mg/dl - an increase of greater than 60%. Measurement of total cholesterol showed an increase due to the increase of the HDL-cholesterol fraction. Measurement of triglycerides showed either no change or a slight decrease. The 30-day study with 3-methyl-TTNEB did not indicate development of tolerance to its pharmacological action.

This study demonstrated that 3-methyl-TTNEB increased plasma concentrations of HDL-cholesterol in a dose-dependent manner following either i.p. or p.o. administration.

The effect on circulating apoAI of orally administered 3-methyl-TTNEB was also studied. Male Sprague-Dawley rats were treated daily with 3 mg/kg body weight of 3-methyl-TTNEB for four days. Serum samples were taken and analyzed by Western Blot using antisera specific to rat apoAI. The treatment with 3-methyl-TTNEB resulted in a significant increase in circulating apoAI level.

These studies demonstrate that treatment with an RXR specific compound such as 3-methyl-TTNEB increases plasma concentrations of apoAI/HDL cholesterol. Since such animal studies are an accepted predictor of human response, it would therefore be expected that such compounds could be used to therapeutically increase HDL-cholesterol in patients who either have, or are at risk for, atherosclerosis.

Additional *in vitro* studies were also performed utilizing the co-transfection assay previously described within this application to demonstrate the effect of RXR-selective ligands on regulation of transcription of apoA1, as described below.

This work focused on studying the transcriptional properties of the retinoid receptors RAR and RXR on a reporter molecule (*e.g.*, luciferase) under control of a basal promoter containing the RXR response element from the apoA1 gene ("A" site). Plasmid constructs coding for the various receptors were transfected into a human hepatocyte cell line (HepG-2) along with the reporter plasmid. Reporter plasmids contained multimers of the apoA1 "A" site (-214 to -192 relative to transcription start site) shown to bind RXR. Widom *et.al., Mol. Cell. Biol.* 12:3380-89 (1992); Ladias & Karathanasis, *Science 251:561-65* (1991). After transfection, treatment, harvest, and assay, the data obtained was normalized to transfected beta-galactosidase activity so as to control for transfection efficiency. The results demonstrated activation in the system with the RXR-specific ligands 3-methyl-TTNCB and 3-methyl-TTNEB in a concentration-dependent fashion, demonstrating that the RXR specific ligands could regulate the transcriptional properties via the "A" site from the apoAI gene. These compounds had no effect when RAR was used in the transfection, demonstrating receptor specific. ity. The transcriptional regulation by RXR was dependent on the presence of the hormone response element.

These *in vivo* and *in vitro* studies demonstrate that the RXR-selective compounds of this invention can be used to elevate apoA1/HDL cholesterol and in the therapeutic treatment of related cardiovascular disorders.

Regarding the modulation of programmed cell death (apoptosis), the retinoid compounds of this invention have been demonstrated to induce apoptosis in particular cell types including leukemic cells and squamous epithelial carcinomas. Normally in cells there is a fine balance between cellular processes of proliferation, differentiation, and cell death, and compounds which affect this balance may be used to treat certain cancers. Specifically, the ability of 3-methyl-TTNEB to induce differentiation, inhibit proliferation, and induce apoptosis in an acute promyelucytic leukemia cell line, HL60, was studied. Cellular proliferation was measured by a thymidine incorporation assay (Shrivastav *et al., Cander Res.,* 40:4438 (1980)), and 3-methyl-TTNEB was found to have no effect on cellular proliferation. This contrasts all-trans-retinoic acid, which inhibits thymidine incorporation. Cellular differation was measured by the ability of the

cells to reduce nitroblue tetrazolium (NBT) (Breitman *et al., Proc. Natl. Acad. Sci.*, 77:2936 (1980)), and 3-methyl-TTNEB was found not to induce undue differentiation. The $EC_{50}$ for 3-methyl-TTNEB mediated differentiation was >1000 μM, compared to 2.0μM for all-trans-retinoic acid. However, 3-methyl-TTNEB was found to induce transglutaminase activity in the HL6O cells (Murtaugh *et al., J. Biol. Chem.* 258:11074 (1983)) in a concentration-dependent manner, which correlates with the induction of apoptosis or programmed cell death. It was further found that 3-methyl-TTNEB was able to induce apoptosis as measured by DNA fragmentation and morphological changes. Other retinoid compounds of this invention showed similar results, and similar results were also shown in other cell lines such as squamous epithelial cell lines and ME180 cells, a human cervical carcinoma.

These results show that RXR specific compounds such as 3-methyl-TTNEB induce apoptosis with a minimal direct effect on inhibition of proliferation and differentiation induction. Compounds which are capable of inducing apoptosis have been shown to be effective in cancer chemotherapy (e.g., anti-hormonal therapy for breast and prostate cancer).

In contrast, in another cell type retinoids have been shown to inhibit activation driven T-cell apoptosis and 9-*cis*-retinoic acid was approximately ten fold more potent than all- *trans*-retinoic acids (Ashwell *et al., Proceedings National Academy of Science, Vol. 90, p. 6170-6174* (1993)). These data imply that RXRs are involved in this event. Thus retinoids could be used to block and/or immunomodulate T-cell apoptosis associated with certain disease states *(e.g.,* AIDS).

It has been surprisingly found that the administration of a ligand which has specific activity for RXRs but essentially no activity for RARs, in combination with a ligand that has specific activity for RARS but not RXRs, provides a cellular response at extremely low dosages, dosages at which the ligands individually provide no significant response. Specifically, the concentration-related effect of an RXR-specific ligand and a RAR-specific ligand on proliferation of a myeloma cell line (RPMI 8226) was studied in *in vitro* studies using a thymidine incorporation assay. This assay examines the incorporation of radiolabeled thymidine into DNA, and by determining the ability of a compound to inhibit thymidine incorporation into DNA, provides a measure of cell proliferation. (L.M. Bradley, Selected Methods in Cellular Immunology, Ch. 10.1, pp. 235-38, Mishell & Shiigi (eds.), Freeman & Co., New York, 1980). Compounds which inhibit cell proliferation have well-known utility in the treatment of certain cancers.

As shown previously (Table 2), 3-methyl-TTNEB activates members of the RXR subfamily and has no significant activity for members of the RAR subfamily. Examination of the effects of 3-methyl-TTNEB on the proliferation of myeloma cells show a concentration dependent inhibition of thymidine incorporation. The $IC_{50}$ (the concentration of 3-methyl-TTNEB required to produce 50% inhibition of the maximal response) is $10^{-7}$ M, as shown in Figure 11. Concentrations less than $10^{-8}$ M provide essentially no effect on cell proliferation, as also shown in Figure 11.

It is well known that the compound TTNPB activates members of the RAR subfamily and has no significant activity for members of the RXR subfamily. The compound TTNPB is shown below, and its activity is shown in Table 5.

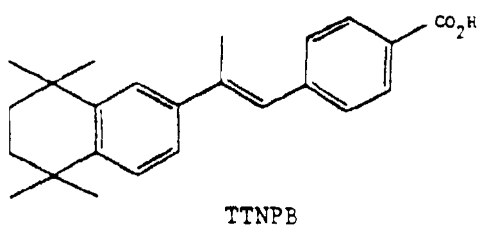

TTNPB

TABLE 5

| | Potency (nM) | Efficacy |
|---|---|---|
| TTNPB | | |
| RXRα | >10,000 | <5% |
| RXRβ | >10,000 | <5% |
| RXRγ | >10,000 | <5% |
| RARα | 52 | 30 |
| RARβ | 4 | 40 |
| RARγ | 0.4 | 50 |

The effect of TTNPB on cell proliferation is shown in Figure 11. The $IC_{50}$ value of TTNPB is about 5 x $10^{-11}$ M, and a concentration of less than $10^{-11}$ M produces essentially no effect on cell proliferation.

However, it has been found that when 3-methyl-TTNEB and TTNPB are present together, each at a concentration where the compound alone produces substantially no anti-proliferative effect, the combination of the two compounds effectively blocks cell proliferation. The combination of the two compounds appears to produce a greater than additive, or synergistic, effect.

For example, as shown in Figure 12, the presence of TTNPB at a concentration of $10^{-11}$ M produces a 9% inhibition on thymidine incorporation. However, combining it with 3-methyl-TTNEB at a concentration of $10^{-8}$ M (which results in no effect on cell proliferation) produces a greatly enhanced inhibitory effect of 49%. Likewise, it has also been found that the inhibitory effect of 3-methyl-TTNEB is greatly increased by the presence of TTNPB at a concentration which alone produces no effect.

Since it is well-known that toxic side effects of compounds such as TTNPB are concentration-dependent, the synergistic effect resulting from combining such RAR-specific compounds with RXR-specific compounds can be expected to permit lower dosages that are efficacious and to therefore reduce toxic side effects. For example, in cancer chemotherapy, use of two such compounds, in combination, at relatively low doses can be expected to produce the desired beneficial effect, while minimizing undesired side effects which result at higher doses of the compounds.

*In vitro* studies utilizing the co-transfection assay have also shown this same synergistic effect. For example, utilizing the co-transfection assay described previously and employing RAR-α and RXR-α and a reporter consisting of the ApoA1 response element "A" site in the context of TKLUC (Ladias & Karathanasis, *Science* 251:561-65 (1991), transfections were performed in HEPG2 cells. In this study, 100 ng of the designated receptor were used and RSVCAT was used as a carrier to keep the amount of RSV promoter constant. All compounds were added at a final concentration of $10^{-7}$ M. The RXR specific compound 3-methyl-TTNEB (Table 2, above) and the RAR specific compound TTNPB (Table 5, above) were utilized. As shown below in Table 6, the relative normalized response observed utilizing the co-transfection assay also demonstrated a synergistic effect when a combination of the two compounds was utilized, compared to the response achieved utilizing the compounds individually.

TABLE 6

| Compound | Reporter Activity (Fold Induction) |
|---|---|
| 3-methyl-TTNEB | 5 |
| TTNPB | 32 |
| 3-methyl-TTNEB + TTNPB | 75 |

As will be discernable to those skilled in the art from the foregoing discussions, the biological response of an RAR selective compound at a given concentration can be synergistically enhanced by combining the compound with an RXR selective compound. Similarly, the biological response of an RXR selective compound can be enhanced by combining the compound with an RAR selective compound. Thus, it becomes possible to achieve a desirable biological response, using a combination of RAR and RXR selective compounds, at lower concentrations than would be the case using the compounds alone. Among the advantages provided by such combinations of RAR and RXR selective compounds are desirable therapeutic effects with fewer side effects. In addition, novel effects that are not obtainable with either agent alone may be achieved by combinations of RAR and RXR selective compounds.

It has been further demonstrated that RXR-specific compounds also synergistically enhance the response of other hormonal systems. Specifically, peroxisome proliferator-activated receptor (PPAR) is a member of the intracellular receptor super family that plays a role in the modulation of lipid homeostasis. PPAR has been shown to be activated by amphipathic carboxylates, such as clofibric acid, and gemfibrizol. These agents, called peroxisome proliferators, have been used in man as hypo-lipidemic agents. The addition of 9-cis-retinoic acid (a retinoid ligand which activates both RAR and RXR receptors) and clofibric acid to HepG2 cells transfected with RXRα and PPAR expression plasmids, results in the activation of receptor gene which was greater than the sum of the activation with each ligand separately. (Kliewer *et al., Nature* 358:771 (1992)). Similarly, when the above two receptors were co-transfected into HepG2 cells, the addition of both an RXR-specific ligand (3-methyl-TTNEB) and clofibric acid was found to produce a greater than additive response as determined by activation of a target reporter gene, as shown below in Table 7.

TABLE 7

| Compound | Normalized Response (%) |
|---|---|
| clofibric Acid | 100 |
| 3-methyl-TTNEB | 90 |

TABLE 7 (continued)

| Compound | Normalized Response (%) |
|---|---|
| clofibric acid + 3-methyl-TTNEB | 425 |

A similar synergistic effect was observed with RXR and RXR-specific ligands and the Vitamin D receptor (VDR) and its cognate ligands. When RXRβ and VD receptors were co-transfected into CV-1 cells containing a hormone response element, the addition of RXR selective 3-methyl-TTNCB and 1,25-dihydroxy-vitamin D (1,25-D) produced a greater than additive response than was observed for each of the individual ligands, as shown below in Table 8.

TABLE 8

| Compound (%) | Normalized Response |
|---|---|
| 1,25-D | 100 |
| 3-Methyl-TTNCB | 13 |
| 1,25-D + 3-methyl-TTNCB | 190 |

As shown, the above results indicate that each pair of receptors (RXRα/PPAR and RXRβ/VDR, respectively), in the presence of ligands known to specifically activate their respective receptors, are capable of producing a synergistic response. The results indicate that the response of a single agent can be enhanced by the combination of the two agents, or that comparable biological or therapeutic responses can be achieved by use of lower doses of such agents in combination.

The observation that RXR-specific ligands are able to act synergistically with RAR ligands, PPAR ligands, and Vitamin D ligands indicates that RXR-specific ligands have usefulness not only as single therapeutic agents but also in combination therapy to obtain enhanced biological or therapeutic response by the addition of the RXR-specific ligand. Such combination therapy also may provide an added benefit of decreasing the side effects associated with the primary agent by employing lower doses of that agent. For example, use of Vitamin D or a related Vitamin D receptor ligand in conjunction with an RXR selective compound for the treatment of a variety of disorders including skin diseases (acne, psoriasis), hyperproliferative disorders (benign and malignant cancers) and disorders of calcium homeostasis may decrease the adverse side effects associated with Vitamin D therapy alone.

As a further example, the RXR-specific compounds of this invention have been demonstrated *in vitro* to act synergistically with compounds which affect cellular proliferation, such as Interferon. Specifically, the growth properties of two human tumor cell lines (ME180, a squamous cell carcinoma, and RPMI18226, a multiple myeloma) were monitored in the presence of the compound 3-methyl-TTNEB alone and in combination with Interferonα2b, utilizing standard cell culture procedures. The effects on growth of these cells were monitored by evaluation of cell number, and also by evaluation of growth in semisolid medium for the RPMI18226 cell line. Both 3-methyl-TTNEB and Interferonα2b were found to inhibit cell growth in a concentration-dependent manner, and each alone to produce a significant depression in cell proliferation. In addition, when the cells were treated with both compounds, an additive or a greater than additive effect on the depression in cell proliferation was observed. Treatment with other chemotherapeutic agents including anti-proliferative agents and/or cell-cycle modulators (*e.g.*, methotrexate, fluorouracil (5-FU, ARA-C, etc.) in combination with RXR-specific compounds would be expected to produce similar results. The enhanced anti-proliferative effect can be expected to permit lower therapeutic doses in treatment of proliferative disorders, such as squamous cell and other carcinomas.

In addition, combination therapy could allow the use of lower doses of these compounds to achieve a comparable beneficial effect along with fewer side effects/ toxic effects, thereby enhancing the therapeutic index of the therapy. The therapeutic index is defined as the ratio of efficacy to toxicity of a compound.

Since RXR is known to form heterodimers with various members of the intracellular receptor super family, it can be expected that the synergistic response observed with use of RXR-selective ligands may be achieved with other receptors with which heterodimers are formed. These include PPARs, RARs, Vitamin D, thyroid hormone receptors, HNF4, the COUP family of receptors, as referenced above, and other as yet unidentified members of the intracellular super family of receptors.

As will be further discernible to those skilled in the art, the compounds disclosed above can be readily utilized in pharmacological applications where selective retinoid receptor activity is desired, and where it is desired to minimize cross reactivities with other related intracellular receptors. *In vivo* applications of the invention include administration of the disclosed compounds to mammalian subjects, and in particular to humans.

The compounds of the present invention are small molecules which are relatively fat soluble or lipophilic and enter the cell by passive diffusion across the plasma membrane. Consequently, these ligands are well suited for administration orally and by injection, as well as topically. Upon administration, these ligands can selectively activate retinoid X receptors, and thereby selectively modulate processes mediated by these receptors.

The pharmaceutical compositions of this invention are prepared in conventional dosage unit forms by incorporating an active compound of the invention, or a mixture of such compounds, with a nontoxic pharmaceutical carrier according to accepted procedures in a nontoxic amount sufficient to produce the desired pharmacodynamic activity in a mammalian and in particular a human subject. Preferably, the composition contains the active ingredient in an active, but nontoxic, amount selected from about 5 mg to about 500 mg of active ingredient per dosage unit. This quantity depends on the specific biological activity desired and the condition of the patient.

The pharmaceutical carrier or vehicle employed may be, for example, a solid or liquid. A variety of pharmaceutical forms can be employed. Thus, when using a solid carrier, the preparation can be plain milled, micronized in oil, tableted, placed in a hard gelatin or enteric-coated capsule in micronized powder or pellet form, or in the form of a troche, lozenge, or suppository. When using a liquid carrier, the preparation can be in the form of a liquid, such as an ampule, or as an aqueous or nonaqueous liquid suspension. For topical administration, the active ingredient may be formulated using bland, moisturizing bases, such as ointments or creams. Examples of suitable ointment bases are petrolatum, petrolatum plus volatile silicones, lanolin, and water in oil emulsions such as Eucerin (Beiersdorf). Examples of suitable cream bases are Nivea Cream (Beiersdorf), cold cream (USP), Purpose Cream (Johnson & Johnson) hydrophilic ointment (USP), and Lubriderm (Warner-Lambert).

Illustrative pharmacological composition formulations are as follows.

Hard gelatin capsules are prepared using the following ingredients:

| | Quantity (mg/capsule) |
|---|---|
| 3-methyl-TTNCB | 140 |
| Starch, dried | 100 |
| Magnesium stearate | 10 |
| Total | 250 mg |

The above ingredients are mixed and filled into hard gelatin capsules in 250 mg quantities.

A tablet is prepared using the ingredients below:

| | Quantity (mg/tablet) |
|---|---|
| 3-methyl-TTNCB | 140 |
| Cellulose, microcrystalline | 200 |
| Silicon dioxide, fumed | 10 |
| Stearic acid | 10 |
| Total | 360 mg |

The components are blended and compressed to form tablets each weighing 360 mg.

Tablets, each containing 60 mg of active ingredient, are made as follows:

| | Quantity (mg/tablet) |
|---|---|
| 3-methyl-TTNCB | 60 |
| Starch | 45 |
| Cellulose, microcrystalline | 35 |
| Polyvinylpyrrolidone (PVP) (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50°C and passed through a No. 18 mesh U.S. sieve. The SCMS, magnesium stearate, and talc, previously passed through a No. 60 mesh U.S. sieve, are then added to the granules which, after mixing, are compressed on a tablet machine to yield tablets each weighing 150 mg.

Suppositories, each containing 225 mg of active ingredient, may be made as follows:

| | |
|---|---|
| 3-methyl-TTNCB | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of normal 2g capacity and allowed to cool.

An intravenous formulation may be prepared as follows:

| | |
|---|---|
| 3-methyl-TTNCB | 100 mg |
| Isotonic saline | 1,000 ml |
| Glycerol | 100 ml |

The compound is dissolved in the glycerol and then the solution is slowly diluted with isotonic saline. The solution of the above ingredients is then administered intravenously at a rate of 1 ml per minute to a patient.

The compounds of this invention also have utility when labeled as ligands for use in assays to determine the presence of RXRs. They are particularly useful due to their ability to selectively bond to members of the RXR subfamily and can therefore be used to determine the presence of RXR isoforms in the presence of other related receptors.

Due to the selective specificity of the compounds of this invention for retinoid X receptors, these compounds can also be used to purify samples of retinoid X receptors *in vitro.* Such purification can be carried out by mixing samples containing retinoid X receptors with one of more of the bicyclic derivative compounds disclosed so that the compound (ligand) binds to the receptor, and then separating out the bound ligand/receptor combination by separation techniques which are known to those of skill in the art. These techniques include column separation, filtration, centrifugation, tagging and physical separation, and antibody complexing, among others.

## Claims

1. A compound having the formula:

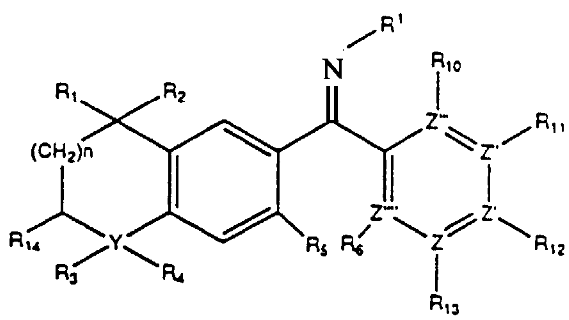

or

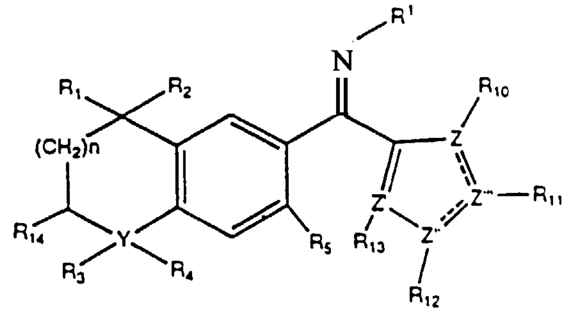

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;
$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' represents HO, NC, $(R_7R_8)N$, $R_{17}O$ or $R_{17}$;
$R_5$ represents hydrogen, a lower alkyl having 1-4 carbons, halogen, nitro, $OR_7$, $SR_7$, $NR_7R_8$, or $(CF)_nCF_3$, with the proviso that if together $R_6$, $R_{10}$, $R_{11}$, $R_{12}$ and $R_{13}$ are all hydrogen, and Z, Z', Z", Z"', and Z"" are all carbon, or if R' represents OH, then $R_5$ is not hydrogen;
$R_6$, $R_{10}$, $R_{11}$, $R_{12}$, $R_{13}$ each independently represent hydrogen, a lower alkyl having 1-4 carbons, halogen, nitro, $OR_7$, $SR_7$, $NR_7R_8$ or $(CF)_nCF_3$, and exist only if the Z, Z', Z", Z"', or Z"" from which it originates is C, or each independently represent hydrogen or a lower alkyl having 1-4 carbons if the Z, Z', Z", Z"', or Z"" from which it originates is N in a five-membered ring, and where one of $R_6$, $R_{10}$, $R_{11}$, $R_{12}$ or $R_{13}$ is X;
$R_7$ and $R_8$ each independently represents hydrogen or a lower alkyl having 1-6 carbons;
$R_9$ represents a lower alkyl having 1-4 carbons, phenyl, aromatic alkyl, or q-hydroxyphenyl, q-bromophenyl, q-chlorophenyl, q-florophenyl, or q-iodophenyl, where q=2-4;
$R_{14}$ represents hydrogen, a lower alkyl having 1-4 carbons, oxo, hydroxy, acyl having 1-4 carbons, halogen, thiol, or thioketone;
$R_{17}$ represents hydrogen, lower alkyl having 1-8 carbons, alkenyl (including halogen, acyl, $OR_7$ and $SR_7$ substituted alkenes), $R_9$, alkyl carboxylic acid (including halogen, acyl, $OR_7$ and $SR_7$ substituted alkyls), alkenyl carboxylic acid (including halogen, acyl, $OR_7$ and $SR_7$ substituted alkenes), alkyl amines (including halogen, acyl, $OR_7$ and $SR_7$ substituted alkyls), and alkenyl amines (including halogen, acryl, $OR_7$ and $SR_7$ substituted alkenes);
X is COOH, tetrazole, $PO_3H$, $SO_3H$, CHO, $CH_2OH$, $CONH_2$, COSH, $COOR_9$, $COSR_9$, $CONHR_9$, or COOW where W is a pharmaceutically acceptable salt, and where X can originate from any C or N on the ring, provided however that X cannot be COOH, CHO, $CH_2OH$, $CONH_2$, $COOR_9$, or COOW when X originates from a C in the 2 or 6 position on the ring;
Z, Z', Z", Z"' and Z"", each independently, represent C, S, O, N, or a pharmaceutically acceptable salt, but is not O or S if attached by a double bond to another such Z or if attached to another such Z which is O or S, and is not N if attached by a single bond to another such Z which is N;
n = 0-3; and

the dashed lines in the second structure shown depict optional double bonds.

**2.** A compound as claimed in claim 1 selected from the group consisting of

ethyl-4-[(3,5,5,8,8-pentamethyl-5,6,7,8-tetrahydro-2-naphthyl)carbonyl]benzoate-oxime,
4-[(3-bromo-5,5,8,8-tetramethyl-5,6,7,8-tefrahydro-2-napthyl)carbonyl]benzoic acid oxime,
2-[(3,5,5,8,8-pentamethyl-5,6,7,8-tetrahydro-2-naphthyl)carbonyl]pyridine-5-carboxylic acid oxime,
ethyl-4-[(3,5,5,8,8-pentamethyl-5,6,7,8-naphthyl)carbonyl]benzoate methyloxime, and
2-[(3,5,5,8,8-pentamethyl-5,6,7,8-tetrahydro-2-naphthyl)carbonyl]pyridine-5-carboxylic acid methyloxime.

**3.** 4-[(3,5,5,8,8-pentamethyl-5,6,7,8-tetrahydro-2-naphthyl)carbonyl] benzoic acid oxime.

**4.** 4-[(3,5,5,8,8-pentamethyl-5,6,7,8-tetrahydro-2-naphthyl) carbonyl] benzoic acid methyloxime.

**5.** A compound as claimed in claim 1 selected from the group consisting of

4-[(3,5,5,8,8-pentamethyl-5, 6,7,8-tetrahydro-2-naphthyl)carbonyl] benzoic acid butyloxime,
4-[(3,5,5,8,8-pentamethyl-5,6,7,8-tetrahydro-2-naphthyl)carbonyl] benzoic acid propyloxime,
4-[(3,5,5,8,8-pentamethyl-5,6,7,8-tetrahydro-2-naphthyl)carbonyl] benzoic acid cyanoimine,
4-[(3,5,5,8,8-pentamethyl-5,6,7,8-tetrahydro-2-naphthyl)carbonyl] benzoic acid allyloxime,
4-[(3,5,5,8,8-pentamethyl-5,6,7,8-tetrahydro-2-naphthyl)carbonyl] benzoic acid 4-(3-methyl but-2-enoic acid) oxime, and
4-[(3,5,5,8,8-pentamethyl-5,6,7,8-tetrahydro-2-naphthyl)carbonyl] benzoic acid 1-amino ethyl oxime.

**6.** A pharmaceutical composition comprising in a pharmaceutically acceptable vehicle suitable for enteral, parenteral, or topical administration, one or more compounds as claimed in any one of claims 1 to 5.

**7.** A compound as claimed in any one of claims 1 to 5 for administration to a mammalian subject to modulate a process mediated by one or more Retinoid X Receptors.

**8.** A compound as claimed in claim 7 for use in modulating a process mediated by Retinoid X Receptors selected from Retinoid X Receptor-alpha, Retinoid X Receptor-beta and Retinoid X Receptor-gamma.

**9.** A compound as claimed in claim 7 or claim 8 for use in modulation of lipid metabolism, modulation of skin-related processes, modulation of malignant cell development, modulation of premalignant lesions, or modulation of programmed cell death.

**10.** A compound as claimed in claim 9 for use in in vivo enhancement of programmed cell death.

**11.** A compound as claimed in claim 9 for use in in vivo inhibition of programmed cell death.

**12.** A compound as claimed in claim 7 or claim 8 for use in modulating *in vivo* cellular growth and differentiation, or *in vivo* limb morphogenesis.

**13.** Use of a compound as claimed in claim 1 to modulate *in vitro* cellular growth and differentiation.

**14.** A compound as claimed in any one of claims 1 to 5 for use in treating a mammalian subject requiring Retinoid X Receptor therapy.

**15.** A compound as claimed in any one of claims 1 to 5 for use in increasing the plasma concentration of high density lipoprotein in a mammalian subject.

**16.** A method for determining the presence of one or more Retinoid X Receptors comprising combining a compound as claimed in any one of claims 1 to 5 with a sample containing one or more unknown receptors and determining whether said compound binds to any receptor in said sample.

**17.** A method of purifying Retinoid X Receptors comprising combining a compound as claimed in any one of claims 1 to 5 with a sample containing one or more said Retinoid X Receptors, allowing said compound to bind with Retinoid X Receptors, and separating out the bound combination of said compound and Retinoid X Receptor.

**18.** A pharmaceutical composition comprising in a pharmaceutically acceptable vehicle for enteral, parenteral, or topical administration a compound as claimed in any one of claims 1 to 5 which selectively activates Retinoid X Receptors in preference to Retinoic Acid Receptors, in combination with a second compound which selectively activates one or more intracellular receptors other than Retinoid X Receptors.

**19.** A pharmaceutical composition as claimed in claim 18 wherein said second compound selectively activates Retinoic Acid Receptors in preference to Retinoid X Receptors.

**20.** A composition as claimed in claim 18 for use in modulating in a mammalian subject a process mediated by intracellular receptors, the physiological effect produced by said composition when administered at a given concentration for said use being greater than the additive effect achieved utilizing each said compound alone at the same concentration.

**21.** A composition as claimed in claim 20 wherein second compound selectively activates Retinoic Acid Receptors in preference to Retinoid X Receptors.

**22.** A composition as claimed in claim 20 for use in modulation of lipid metabolism, modulation of skin-related processes, modulation of malignant cell development, modulation of premalignant lesions, or modulation of programmed cell death.

**23.** A composition as claimed in claim 20 or claim 21 which is capable of being administered at a concentration at which neither said first nor second compound would alone produce a significant therapeutic response.

**24.** A composition as claimed in claim 20 wherein said second compound activates peroxisome proliferator activated receptors.

**25.** A composition as claimed in claim 20 wherein said second compound activates Vitamin D receptors.

**26.** A composition as claimed in claim 20 wherein said second compound activates thyroid hormone receptors, HNF4 receptors, or members of the COUP family of receptors.

**Patentansprüche**

**1.** Verbindung mit der Formel:

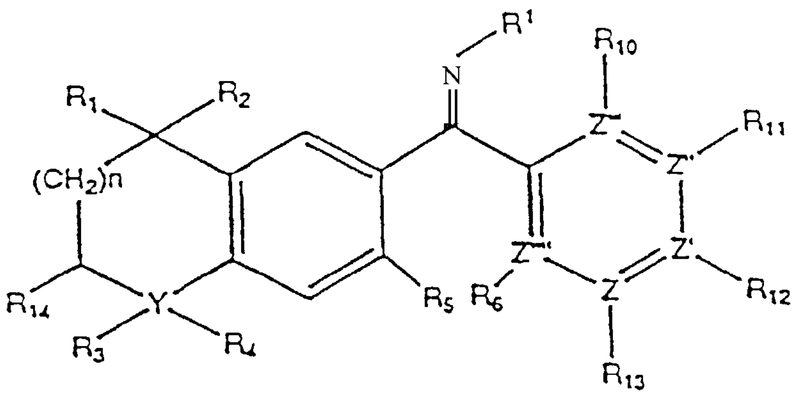

oder

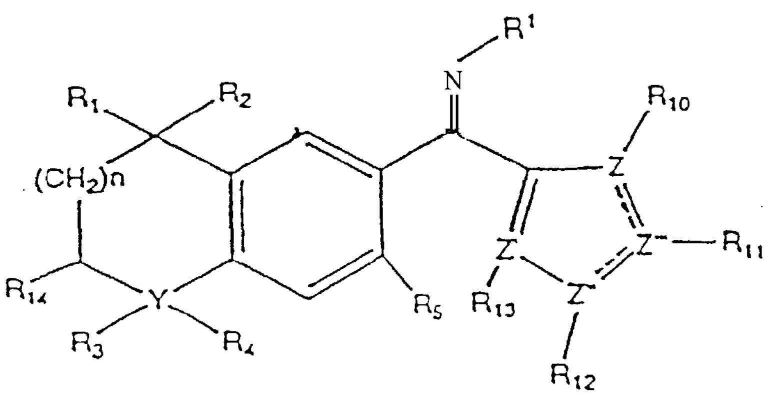

,

worin

$R_1$ und $R_2$, jeweils unabhängig, Wasserstoff oder Niederalkyl oder -acyl mit 1-4 Kohlenstoffatomen repräsentieren;

Y für C, O, S, N, CHOH, CO, SO, $SO_2$ oder ein pharmazeutisch annehmbares Salz steht;

$R_3$ Wasserstoff oder Niederalkyl mit 1-4 Kohlenstoffatomen repräsentiert, wobei Y C oder N ist;

$R_4$ Wasserstoff oder Niederalkyl mit 1-4 Kohlenstoffatomen repräsentiert, wobei Y C ist, aber $R_4$ nicht exisitiert, wenn Y N ist, und weder $R_3$ noch $R_4$ existieren, wenn Y S, O, CHOH, CO, SO oder $SO_2$ ist;

R' für HO, NC, $(R_7R_8)N$, $R_{17}O$ oder $R_{17}$ steht;

$R_5$ Wasserstoff, ein Niederalkyl mit 1-4 Kohlenstoffatomen, Halogen, Nitro, $OR_7$, $SR_7$, $NR_7R_8$ oder $(CF)_nCF_3$ repräsentiert, mit der Maßgabe, daß wenn $R_6$, $R_{10}$, $R_{11}$, $R_{12}$ und $R_{13}$ alle Wasserstoff sind und Z, Z', Z", Z"' und Z"" alle Kohlenstoff sind, oder wenn R' für OH steht, $R_5$ dann nicht Wasserstoff ist;

$R_6$, $R_{10}$, $R_{11}$, $R_{12}$, $R_{13}$ jeweils unabhängig Wasserstoff, ein Niederalkyl mit 1-4 Kohlenstoffatomen, Halogen, Nitro, $OR_7$, $SR_7$, $NR_7R_8$, oder $(CF)_nCF_3$ repräsentieren und nur existieren, wenn das Z, Z', Z", Z"' oder Z"", von denen es ausgeht, C ist, oder jeweils unabhängig Wasserstoff oder ein Niederalkyl mit 1-4 Kohlenstoffatomen repräsentieren, wenn das Z, Z', Z", Z"' oder Z"", von denen es ausgeht, N in einem fünfgliedrigen Ring ist, und wobei eines von $R_6$, $R_{10}$, $R_{11}$, $R_{12}$ oder $R_{13}$ X ist;

$R_7$ und $R_8$ jeweils unabhängig Wasserstoff oder ein Niederalkyl mit 1-6 Kohlenstoffatomen repräsentieren;

$R_9$ ein Niederalkyl mit 1-4 Kohlenstoffatomen, Phenyl, aromatisches Alkyl, oder q-Hydroxyphenyl, q-Bromphenyl, q-Chlorphenyl, q-Fluorphenyl oder q-Iodphenyl repräsentiert, wobei q = 2-4;

$R_{14}$ Wasserstoff, ein Niederalkyl mit 1-4 Kohlenstoffatomen, Oxo, Hydroxy, Acyl mit 1-4 Kohlenstoffatomen, Halogen, Thiol oder Thioketon repräsentiert;

$R_{17}$ Wasserstoff, ein Niederalkyl mit 1-8 Kohlenstoffatomen, Alkenyl (einschließlich Halogen-, Acyl-, $OR_7$- und $SR_7$-substituierten Alkenen), $R_9$, Alkylcarbonsäure (einschließlich Halogen-, Acyl-, $OR_7$- und $SR_7$-substituierten Alkylen), Alkenylcarbonsäure (einschließlich Halogen-, Acyl-, $OR_7$- und $SR_7$-substituierten Alkenen), Alkylamine (einschließlich Halogen-, Acyl-, $OR_7$- und $SR_7$-substituierten Alkylen) und Alkenylamine (einschließlich Halogen-, Acryl-, $OR_7$- und $SR_7$-substituierten Alkenen) repräsentiert;

X COOH, Tetrazol, $PO_3H$, $SO_3H$, CHO, $CH_2OH$, $CONH_2$, COSH, $COOR_9$, $COSR_9$, $CONHR_9$ oder COOW ist, wobei W ein pharmazeutisch annehmbares Salz ist, und wobei X von jedwedem C oder N auf dem Ring ausgehen kann, allerdings mit der Voraussetzung, daß X nicht COOH, CHO, $CH_2OH$, $CONH_2$, $COOR_9$ oder COOW sein kann, wenn X von einem C in der 2- oder 6-Position auf dem Ring ausgeht;

Z, Z', Z", Z"' und Z"" jeweils unabhängig C, S, O, N oder ein pharmazeutisch annehmbares Salz repräsentiert, aber nicht O oder S ist, wenn durch eine Doppelbindung an ein anderes solches Z verknüpft vorliegend oder wenn an ein anderes solches Z, das O oder S ist, verknüpft vorliegend, und nicht N ist, wenn durch eine Einfachbindung an ein anderes solches Z verknüpft vorliegend, das N ist;

n = 0-3 ist; und

die gestrichelten Linien in der zweiten gezeigten Struktur optionale Doppelbindungen verbildlichen.

**2.** Verbindung, wie beansprucht in Anspruch 1, gewählt aus der Gruppe, bestehend aus

Ethyl-4-[(3,5,5,8,8-pentamethyl-5,6,7,8-tetrahydro-2-naphthyl)carbonyl]benzoatoxim,
4-[(3-Brom-5,5,8,8-tetramethyl-5,6,7,8-tetrahydro-2-naphthyl)carbonyl]benzoesäureoxim,
2-[(3,5,5,8,8-Pentamethyl-5,6,7,8-tetrahydro-2-naphthyl)carbonyl]pyridin-5-carbonsäureoxim,
Ethyl-4-[(3,5,5,8,8-pentamethyl-5,6,7,8-tetrahydro-2-naphthyl)carbonyl]benzoatmethyloxim, und
2-[(3,5,5,8,8-Pentamethyl-5,6,7,8-tetrahydro-2-naphthyl)carbonyl]pyridin-5-carbonsäuremethyloxim.

3. 4-[(3,5,5,8,8-Pentamethyl-5,6,7,8-tetrahydro-2-naphthyl)carbonyl]benzoesäureoxim.

4. 4-[(3,5,5,8,8-Pentamethyl-5,6,7,8-tetrahydro-2-naphthyl)carbonyl]benzoesäuremethyloxim.

5. Verbindung, wie beansprucht in Anspruch 1, gewählt aus der Gruppe, bestehend aus

4-[(3,5,5,8,8-Pentamethyl-5,6,7,8-tetrahydro-2-naphthyl)carbonyl]benzoesäurebutyloxim,
4-[(3,5,5,8,8-Pentamethyl-5,6,7,8-tetrahydro-2-naphthyl)carbonyl]benzoesäure-propyloxim,
4-[(3,5,5,8,8-Pentamethyl-5,6,7,8-tetrahydro-2-naphthyl)carbonyl]benzoesäurecyanoimin,
4-[(3,5,5,8,8-Pentamethyl-5,6,7,8-tetrahydro-2-naphthyl)carbonyl]benzoesäure-allyloxim,
4-[(3,5,5,8,8-Pentamethyl-5,6,7,8-tetrahydro-2-naphthyl)carbonyl]benzoesäüre-4-(3-methyl-but-2-enonsäure)oxim, und
4-[(3,5,5,8,8-Pentamethyl-5,6,7,8-tetrahydro-2-naphthyl)carbonyl]benzoesäure-l-aminoethyloxim.

6. Pharmazeutische Zusammensetzung, umfassend in einem pharmazeutisch annehmbaren Vehikel, geeignet für enterale, parenterale oder topische Verabreichung, eine oder mehrere Verbindungen, wie beansprucht in mindestens einem der Ansprüche 1 bis 5.

7. Verbindung, wie beansprucht in mindestens einem der Ansprüche 1 bis 5, zur Verabreichung an ein Säugersubjekt, um einen Vorgang zu modulieren, der von einem oder mehreren Retinoid X-Rezeptoren vermittelt wird.

8. Verbindung, wie beansprucht in Anspruch 7, zur Verwendung beim Modulieren eines Vorgangs, der vermittelt wird von Retinoid X-Rezeptoren, gewählt aus Retinoid X-Rezeptor-alpha, Retinoid X-Rezeptor-beta und Retinoid X-Rezeptor-gamma.

9. Verbindung, wie beansprucht in Anspruch 7 oder Anspruch 8, zur Verwendung bei der Modulation des Lipidstoffwechsels, Modulation von mit der Haut zusammenhängenden Vorgängen, Modulation von maligner Zellentwicklung, Modulation von prämalignen Läsionen oder Modulation des programmierten Zelltodes.

10. Verbindung, wie beansprucht in Anspruch 9, zur Verwendung bei der in vivo-Verstärkung des programmierten Zelltodes.

11. Verbindung, wie beansprucht in Anspruch 9, zur Verwendung bei der in vivo-Inhibition des programmierten Zelltodes.

12. Verbindung, wie beansprucht in Anspruch 7 oder Anspruch 8, zur Verwendung bei der Modulierung von in vivo-Zellwachstum und -differenzierung oder in vivo-Gliedmaßen-Morphogenese.

13. Verwendung einer Verbindung, wie beansprucht in Anspruch 1, zur Modulierung von in vitro-Zellwachstum und -differenzierung.

14. Verbindung, wie beansprucht in mindestens einem der Ansprüche 1 bis 5, zur Verwendung bei der Behandlung eines Säugersubjekts, das einer Retinoid X-Rezeptor-Therapie bedarf.

15. Verbindung, wie beansprucht in mindestens einem der Ansprüche 1 bis 5, zur Verwendung bei der Erhöhung der Plasmakonzentration von Hoch-Dichte-Lipoprotein in einem Säugersubjekt.

16. Verfahren zur Bestimmung der Gegenwart von einem oder mehreren Retinoid X-Rezeptoren, umfassend das Vereinigen einer Verbindung, wie beansprucht in mindestens einem der Ansprüche 1 bis 5, mit einer Probe, enthaltend einen oder mehrere unbekannte Rezeptoren, und die Bestimmung, ob die Verbindung an irgendeinen Rezeptor in der Probe bindet.

17. Verfahren zur Reinigung von Retinoid X-Rezeptoren, umfassend das Vereinigen einer Verbindung, wie beansprucht in mindestens einem der Ansprüche 1 bis 5, mit einer Probe, enthaltend einen oder mehrere besagte(n) Retinoid X-Rezeptor(en), wobei zugelassen wird, daß die Verbindung mit Retinoid X-Rezeptoren bindet, und das Abtrennen der gebundenen Kombination von besagter Verbindung und Retinoid X-Rezeptor.

18. Pharmazeutische Zusammensetzung, umfassend in einem pharmazeutisch annehmbaren Vehikel für enterale,

parenterale oder topische Verabreichung, eine Verbindung, wie beansprucht in mindestens einem der Ansprüche 1 bis 5, welche selektiv Retinoid X-Rezeptoren in Bevorzugung gegenüber Retinsäure-Rezeptoren aktiviert, in Kombination mit einer zweiten Verbindung, welche selektiv einen oder mehrere von Retinoid X-Rezeptoren verschiedene intrazelluläre Rezeptoren aktiviert.

**19.** Pharmazeutische Zusammensetzung, wie beansprucht in Anspruch 18, wobei die zweite Verbindung selektiv Retinsäure-Rezeptoren in Bevorzugung gegenüber Retinoid X-Rezeptoren aktiviert.

**20.** Zusammensetzung, wie beansprucht in Anspruch 18, zur Verwendung bei der Modulierung eines durch intrazelluläre Rezeptoren vermittelten Vorgangs in einem Säugersubjekt, wobei der von der Zusammensetzung hervorgerufene physiologische Effekt bei Verabreichung bei einer gegebenen Konzentration für die Verwendung größer ist als der additive Effekt, der bei alleiniger Anwendung jeder Verbindung bei der gleichen Konzentration erzielt wird.

**21.** Zusammensetzung, wie beansprucht in Anspruch 20, wobei die zweite Verbindung selektiv Retinsäure-Rezeptoren in Bevorzugung gegenüber Retinoid X-Rezeptoren aktiviert.

**22.** Zusammensetzung, wie beansprucht in Anspruch 20, zur Verwendung bei der Modulation von Lipidstoffwechsel, Modulation von mit der Haut zusammenhängenden Vorgängen, Modulation von maligner Zellentwicklung, Modulation von prämalignen Läsionen oder Modulation des programmierten Zelltodes.

**23.** Zusammensetzung, wie beansprucht in Anspruch 20 oder Anspruch 21, die dazu in der Lage ist, bei einer Konzentration verabreicht zu werden, bei der weder die erste noch die zweite Verbindung allein eine signifikante therapeutische Antwort erzeugen würden.

**24.** Zusammensetzung, wie beansprucht in Anspruch 20, wobei die zweite Verbindung Peroxisom-Proliferator-aktivierte Rezeptoren aktiviert.

**25.** Zusammensetzung, wie beansprucht in Anspruch 20, wobei die zweite Verbindung Vitamin-D-Rezeptoren aktiviert.

**26.** Zusammensetzung, wie beansprucht in Anspruch 20, wobei die zweite Verbindung Thyroidhormon-Rezeptoren, HNF4-Rezeptoren oder Mitglicder der COUP-Rezeptorenfamilie aktiviert.

**Revendications**

**1.** Composé répondant à la formule :

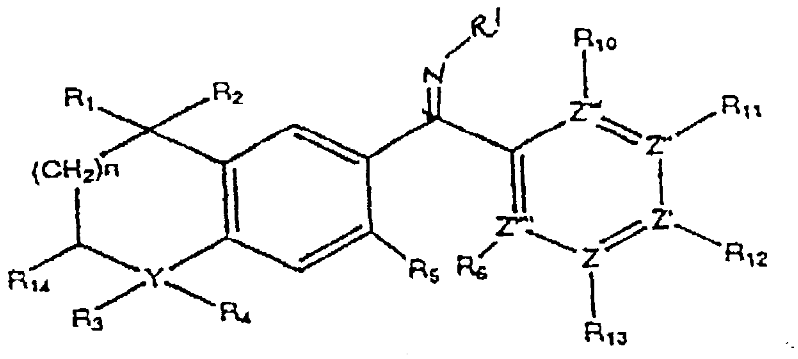

ou

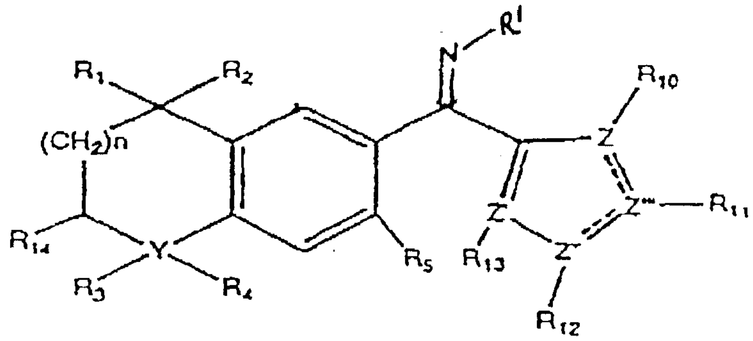

dans laquelle

$R_1$ et $R_2$ représentent, chacun indépendamment, un hydrogène ou un alkyle inférieur ou acyle inférieur comportant 1 à 4 atomes de carbone ;
Y représente C, O, S, N, CHOH, CO, SO, $SO_2$, ou forme un sel pharmaceutiquement acceptable ;
$R_3$ représente un hydrogène ou un alkyle inférieur comportant 1 à 4 atomes de carbone lorsque Y représente C ou N ;
$R_4$ représente un hydrogène ou un alkyle inférieur comportant 1 à 4 atomes de carbone lorsque Y représente C, mais $R_4$ n'existe pas si Y représente N, et ni $R_3$ ni $R_4$ n'existent si Y représente S, O, CHOH, CO, SO, ou $SO_2$ ;
R' représente HO, NC, $(R_7R_8)N$, $R_{17}O$ ou $R_{17}$ ;
$R_5$ représente un hydrogène, alkyle inférieur comportant 1 à 4 atomes de carbone, halogène, nitro, $OR_7$, $SR_7$, $NR_7R_8$, ou $(CF)_nCF_3$, sous réserve que si $R_6$, $R_{10}$, $R_{11}$, $R_{12}$ et $R_{13}$ représentent tous un hydrogène et Z, Z', Z", Z''' et Z'''' représentent tous un carbone, ou si R' représente OH, alors $R_5$ ne représente pas un hydrogène ;
$R_6$, $R_{10}$, $R_{11}$, $R_{12}$, $R_{13}$ représentent, chacun indépendamment, un hydrogène, alkyle inférieur comportant 1 à 4 atomes de carbone, halogène, nitro, $OR_7$, $SR_7$, $NR_7R_8$, ou $(CF)_nCF_3$, et existent seulement si les Z, Z', Z ", Z''' ou Z'''' auxquels ils sont rattachés représentent C, ou représentent, chacun indépendamment, un hydrogène, un alkyle inférieur comportant 1 à 4 atomes de carbone si les Z, Z', Z ", Z''' et Z'''' auxquels ils sont rattachés représentent N dans un cycle à 5 chaînons, et l'un des $R_6$, $R_{10}$, $R_{11}$, $R_{12}$ ou $R_{13}$ représente X ;
$R_7$ et $R_8$ représentent, chacun indépendamment, un hydrogène ou un alkyle inférieur comportant 1 à 6 atomes de carbone ;
$R_9$ représente un alkyle inférieur comportant 1 à 4 atomes de carbone, un phényle, aromatique-alkyle, ou q-hydroxyphényle, q-bromophényle, q-chlorophényle, q-fluorophényle ou q-iodophényle, sachant que q = 2 - 4 ;
$R_{14}$ représente un hydrogène, alkyle inférieur comportant 1 à 4 atomes de carbone, oxo, hydroxy, acyle comportant 1 à 4 atomes de carbone, halogène, thiol ou thiocétone ;
$R_{17}$ représente un hydrogène, alkyle inférieur comportant 1 à 8 atomes de carbone, alcényle (y compris des alcènes substitués par halogène, acyle, $OR_7$ et $SR_7$), $R_9$, acide alkylcarboxylique (y compris avec des alkyles substitués par halogène, acyle, $OR_7$ et $SR_7$), acide alcénylcarboxylique (y compris avec des alcènes substitués par halogène, acyle, $OR_7$ et $SR_7$), alkylamine (y compris avec des alkyles substitués par halogène, acyle, $OR_7$ et $SR_7$), et alcénylamine (y compris avec des alcènes substitués par halogène, un groupe acyle, $OR_7$ et $SR_7$) ;
X représente COOH, tétrazole, $PO_3H$, $SO_3H$, CHO, $CH_2OH$, $CONH_2$, COSH, $COOR_9$, $COSR_9$, $CONHR_9$, ou COOW, où W est un sel pharmaceutiquement acceptable et X peut être fixé sur l'un quelconque des C ou N sur le cycle, étant entendu que X ne peut pas représenter COOH, CHO, $CH_2OH$, $CONH_2$, $COOR_9$, ou COOW lorsque X est fixé sur un C en position 2 ou 6 sur le cycle ;
Z, Z', Z", Z''' et Z'''' représentent, chacun indépendamment, C, S, O, N ou un sel pharmaceutiquement acceptable, mais ne représentent ni O ni S s'ils sont fixés par une double liaison à un autre de ces Z ou s'ils sont fixés à un autre de ces Z qui représente O ou S, et ne représentent pas N s'ils sont fixés par une simple liaison à un autre de ces Z qui représente N ;
n = 0 - 3 ; et

les tirets représentent, dans la seconde structure représentée, des doubles liaisons facultatives.

2. Composé selon la revendication 1 choisi dans le groupe constitué par

le 4-[(3,5,5,8,8-pentaméthyl-5,6,7,8-tétrahydro-2-naphtyl)carbonyl]benzoate d'éthyle-oxime,
l'acide 4-[(3-bromo-5,5,8,8-tétraméthyl-5,6,7,8-tétrahydro-2-naphtyl)carbonyl]benzoïqueoxime,
l'acide 2-[(3,5,5,8,8-pentaméthyl-5,6,7,8-tétrahydro-2-naphtyl)carbonyl]pyridine-5-carboxyliqueoxime,
le 4-[(3,5,5,8,8-pentaméthyl-5,6,7,8-tétrahydro-2-naphtyl)carbonyl]benzoate d'éthyleméthyloxime, et
l'acide 2-[(3,5,5,8,8-pentaméthyl-5,6,7,8-tétrahydro-2-naphtyl)carbonyl]pyridine-5-carboxyliqueméthyloxime.

**3.** Acide 4-[(3,5,5,8,8-pentaméthyl-5,6,7,8-tétrahydro-2-naphtyl)carbonyl]benzoïqueoxime.

**4.** Acide 4-[(3,5,5,8,8-pentaméthyl-5,6,7,8-tétrahydro-2-naphtyl)carbonyl]benzoïqueméthyloxime.

**5.** Composé selon la revendication 1 choisi dans le groupe constitué par

l'acide 4-[(3,5,5,8,8-pentaméthyl-5,6,7,8-tétrahydro-2-naphtyl)carbonyl]benzoïquebutyloxime,
l'acide 4-[(3,5,5,8,8-pentaméthyl-5,6,7,8-tétrahydro-2-naphtyl)carbonyl]benzoïquepropyloxime,
l'acide 4-[(3,5,5,8,8-pentaméthyl-5,6,7,8-tétrahydro-2-naphtyl)carbonyl]benzoïquecyanoimine,
l'acide 4-[(3,5,5,8,8-pentaméthyl-5,6,7,8-tétrahydro-2-naphtyl)carbonyl]benzoïqueallyloxime,
l'acide 4-[(3,5,5,8,8-pentaméthyl-5,6,7,8-tétrahydro-2-naphtyl)carbonyl]benzoïque4-(acide 3-méthyl-but-2-énoïque)oxime, et
l'acide 4-[(3,5,5,8,8-pentaméthyl-5,6,7,8-tétrahydro-2-naphtyl)carbonyl]benzoïque1-aminoéthyloxime.

**6.** Composition pharmaceutique comprenant, dans un véhicule pharmaceutiquement acceptable approprié pour l'administration entérale, parentérale ou topique, un ou plusieurs composés tels que revendiqués dans l'une quelconque des revendications 1 à 5.

**7.** Composé selon l'une quelconque des revendications 1 à 5, destiné à être administré à un sujet mammifère pour la modulation d'un processus dont la médiation est assurée par un ou plusieurs récepteurs X de rétinoïde.

**8.** Composé selon la revendication 7, destiné à être utilisé pour la modulation d'un processus dont la médiation est assurée par des récepteurs X de rétinoïde choisis parmi le récepteur X alpha de rétinoïde, le récepteur X bêta de rétinoïde et le récepteur X gamma de rétinoïde.

**9.** Composé selon la revendication 7 ou la revendication 8, destiné à être utilisé pour la modulation du métabolisme des lipides, la modulation de processus en relation avec la peau, la modulation du développement de cellules malignes, la modulation de lésions prémalignes ou la modulation de la mort programmée des cellules.

**10.** Composé selon la revendication 9, destiné à être utilisé pour l'augmentation *in vivo* de la mort programmée des cellules.

**11.** Composé selon la revendication 9, destiné à être utilisé pour l'inhibition *in vivo* de la mort programmée des cellules.

**12.** Composé selon la revendication 7 ou la revendication 8, destiné à être utilisé dans la modulation de la croissance et de la différenciation des cellules *in vivo,* ou de la morphogénèse limbique *in vivo.*

**13.** Utilisation d'un composé selon la revendication 1 dans la modulation de la croissance et de la différenciation cellulaire *in vitro.*

**14.** Composé selon l'une quelconque des revendications 1 à 5, destiné à être utilisé pour le traitement d'un sujet mammifère nécessitant une thérapie par récepteur X de rétinoïde.

**15.** Composé selon l'une quelconque des revendications 1 à 5, destiné à être utilisé pour augmenter la concentration plasmatique des lipoprotéines de haute densité chez un sujet mammifère.

**16.** Méthode pour déterminer la présence d'un ou plusieurs récepteurs X de rétinoïde comprenant les étapes consistant à combiner un composé tel que revendiqué dans l'une quelconque des revendications 1 à 5 avec un échantillon contenant un ou plusieurs récepteurs inconnus et à déterminer si ledit composé se lie à un quelconque récepteur présent dans ledit échantillon.

**17.** Procédé pour purifier des récepteurs X de rétinoïde comprenant les étapes consistant à combiner un composé tel

que revendiqué dans l'une quelconque des revendications 1 à 5 avec un échantillon contenant un ou plusieurs desdits récepteurs X de rétinoïde, à laisser ledit composé se lier aux récepteurs X de rétinoïde, et à séparer la combinaison liée desdits composé et des récepteurs X de rétinoïde.

**18.** Composition pharmaceutique comprenant, dans un véhicule pharmaceutiquement acceptable pour l'administration entérale, parentérale ou topique, un composé tel que revendiqué dans l'une quelconque des revendications 1 à 5 qui active sélectivement les récepteurs X de rétinoïde de préférence aux récepteurs d'acide rétinoïque, en combinaison avec un second composé qui active sélectivement un ou plusieurs récepteurs intracellulaires autres que les récepteurs X de rétinoïde.

**19.** Composition pharmaceutique selon la revendication 18, dans laquelle ledit second composé active sélectivement les récepteurs d'acide rétinoïque de préférence aux récepteurs X de rétinoïde.

**20.** Composition pharmaceutique selon la revendication 18, destinée à être utilisée pour la modulation, chez un sujet mammifère, d'un processus dont la médiation est assurée par des récepteurs intracellulaires, l'effet physiologique produit par ladite composition, lorsqu'elle est administrée à une concentration donnée pour ladite utilisation, étant supérieur à l'addition des effets obtenus en utilisant chacun desdits composés seuls à la même concentration.

**21.** Composition selon la revendication 20, dans laquelle le second composé active sélectivement les récepteurs d'acide de rétinoïque de préférence aux récepteurs X de rétinoïde.

**22.** Composition selon la revendication 20, destinée à être utilisée pour la modulation du métabolisme des lipides, la modulation de processus en relation avec la peau, la modulation du développement de cellules malignes, la modulation de lésions prémalignes ou la modulation de la mort programmée des cellules.

**23.** Composition selon la revendication 20 ou la revendication 21, qui peut être administrée à une concentration à laquelle ni le premier, ni le second composé, ne donnerai seul une réponse thérapeutique significative.

**24.** Composition selon la revendication 20, dans laquelle ledit second composé active les récepteurs activés par des proliférateurs de péroxysome.

**25.** Composition selon la revendication 20, dans laquelle ledit second composé active les récepteurs de la vitamine D.

**26.** Composition selon la revendication 20, dans laquelle ledit second composé active les récepteurs d'hormone thyroïdienne, les récepteurs HNF4, ou des membres de la famille de récepteurs COUP.

FIG. 1.

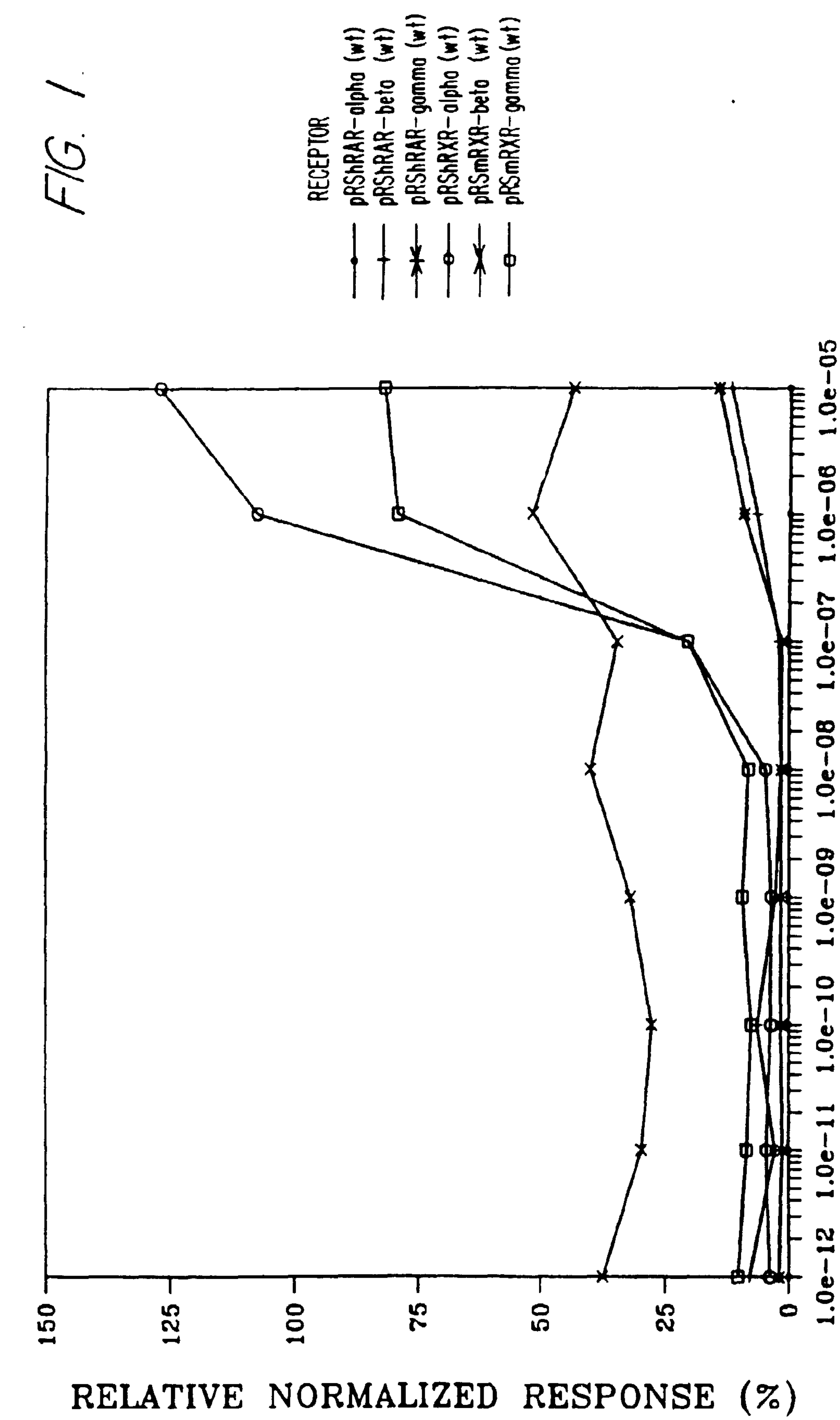
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
RELATIVE NORMALIZED RESPONSE (%)
0
25
50
75
100
125
150
CONCENTRATION (M)
1.0e-12
1.0e-11
1.0e-10
1.0e-09
1.0e-08
1.0e-07
1.0e-06
1.0e-05

FIG. 2

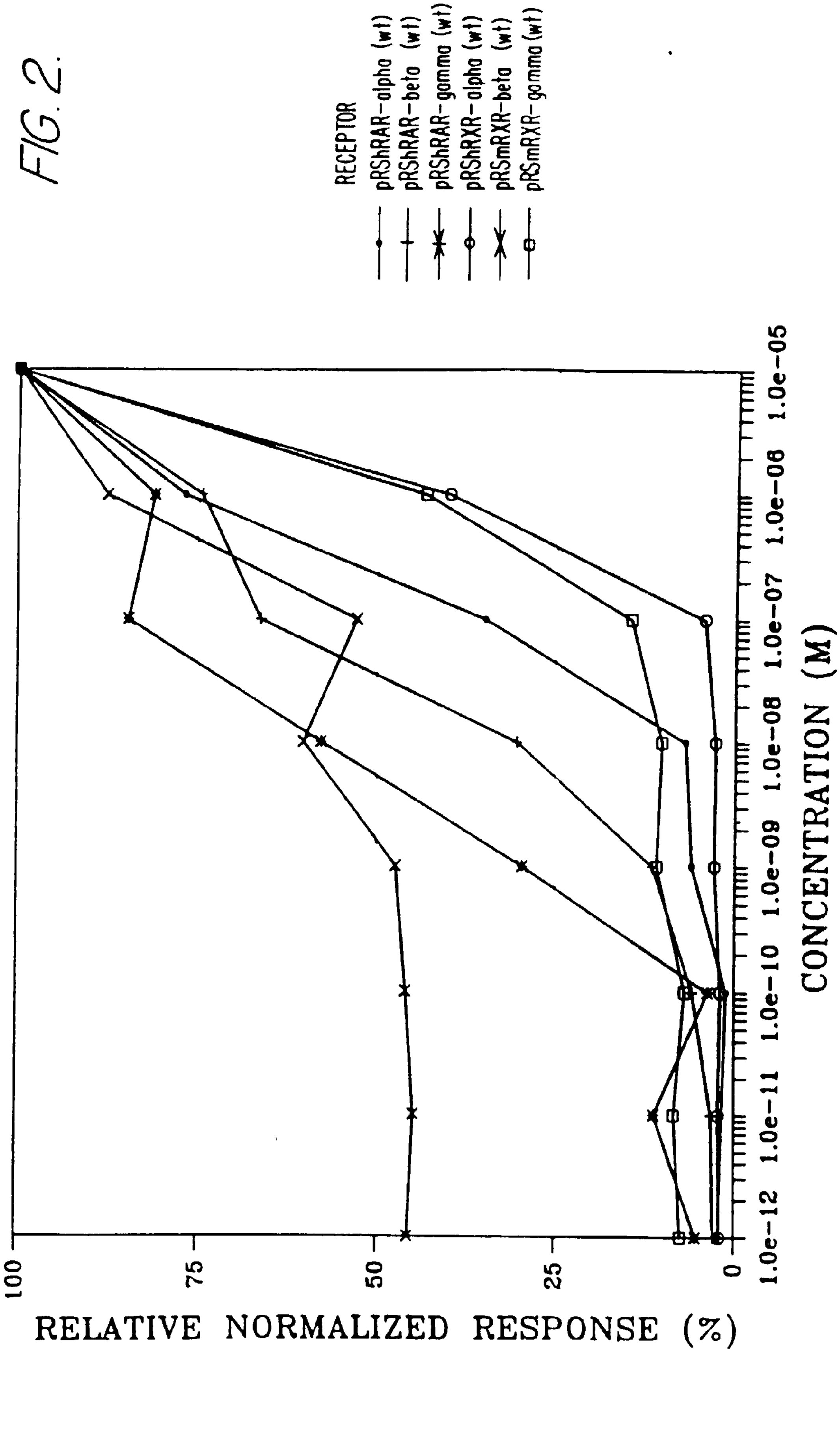
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)

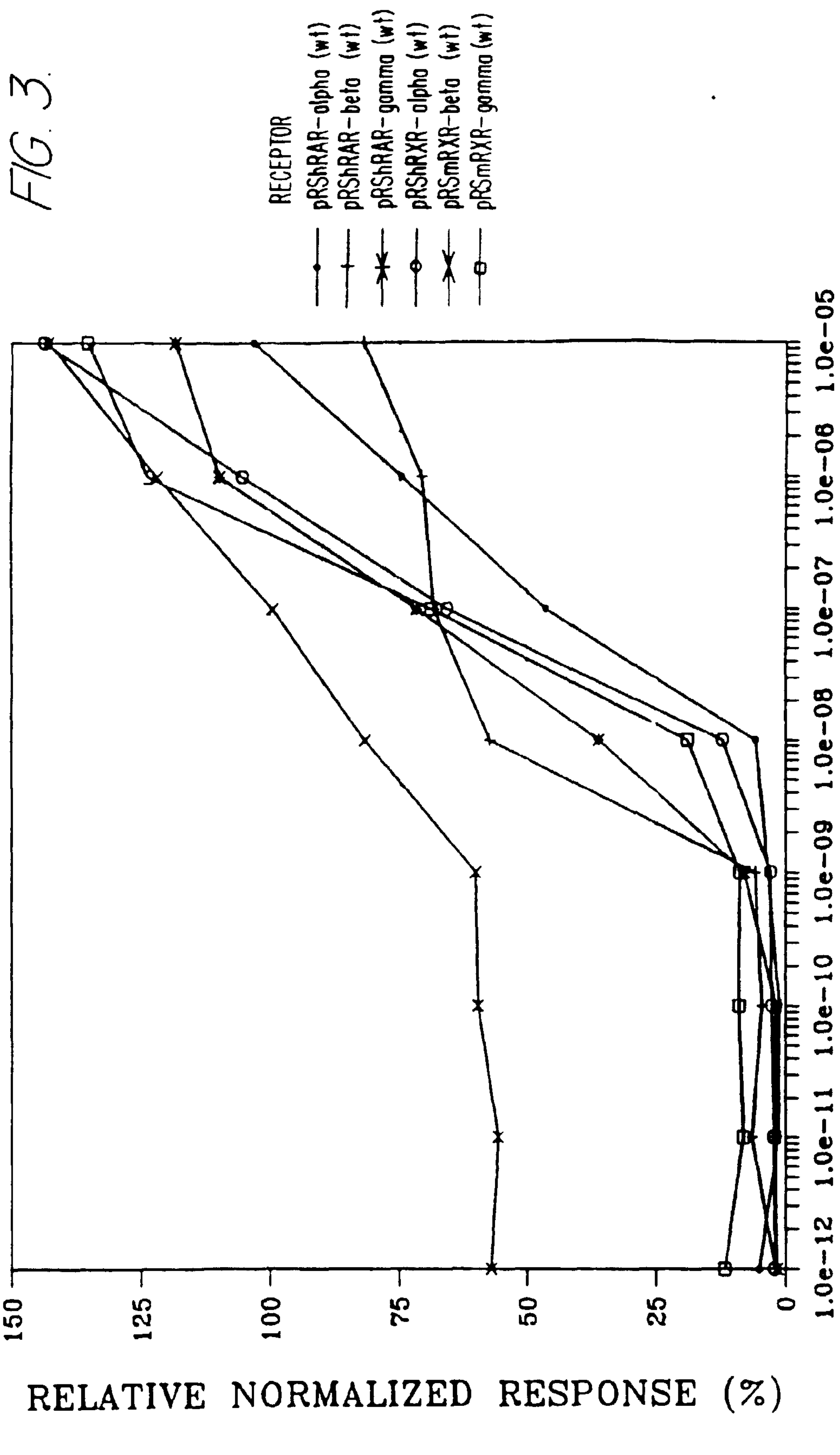
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
RELATIVE NORMALIZED RESPONSE (%)
150
125
100
75
50
25
0
CONCENTRATION (M)
1.0e-12
1.0e-11
1.0e-10
1.0e-09
1.0e-08
1.0e-07
1.0e-06
1.0e-05

FIG. 3

FIG. 4

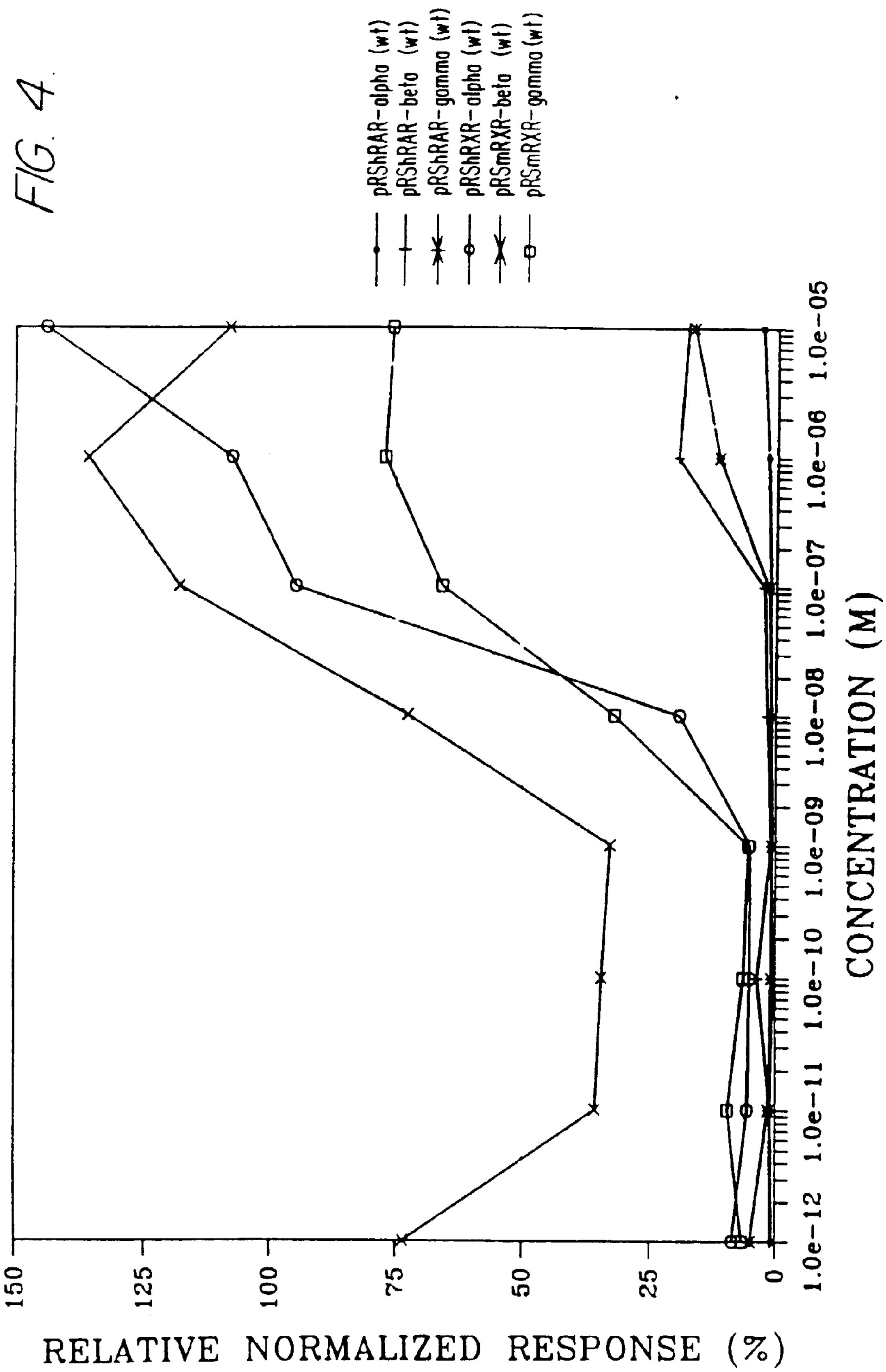
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
150
125
100
75
50
25
0
1.0e-12
1.0e-11
1.0e-10
1.0e-09
1.0e-08
1.0e-07
1.0e-06
1.0e-05
CONCENTRATION (M)
RELATIVE NORMALIZED RESPONSE (%)

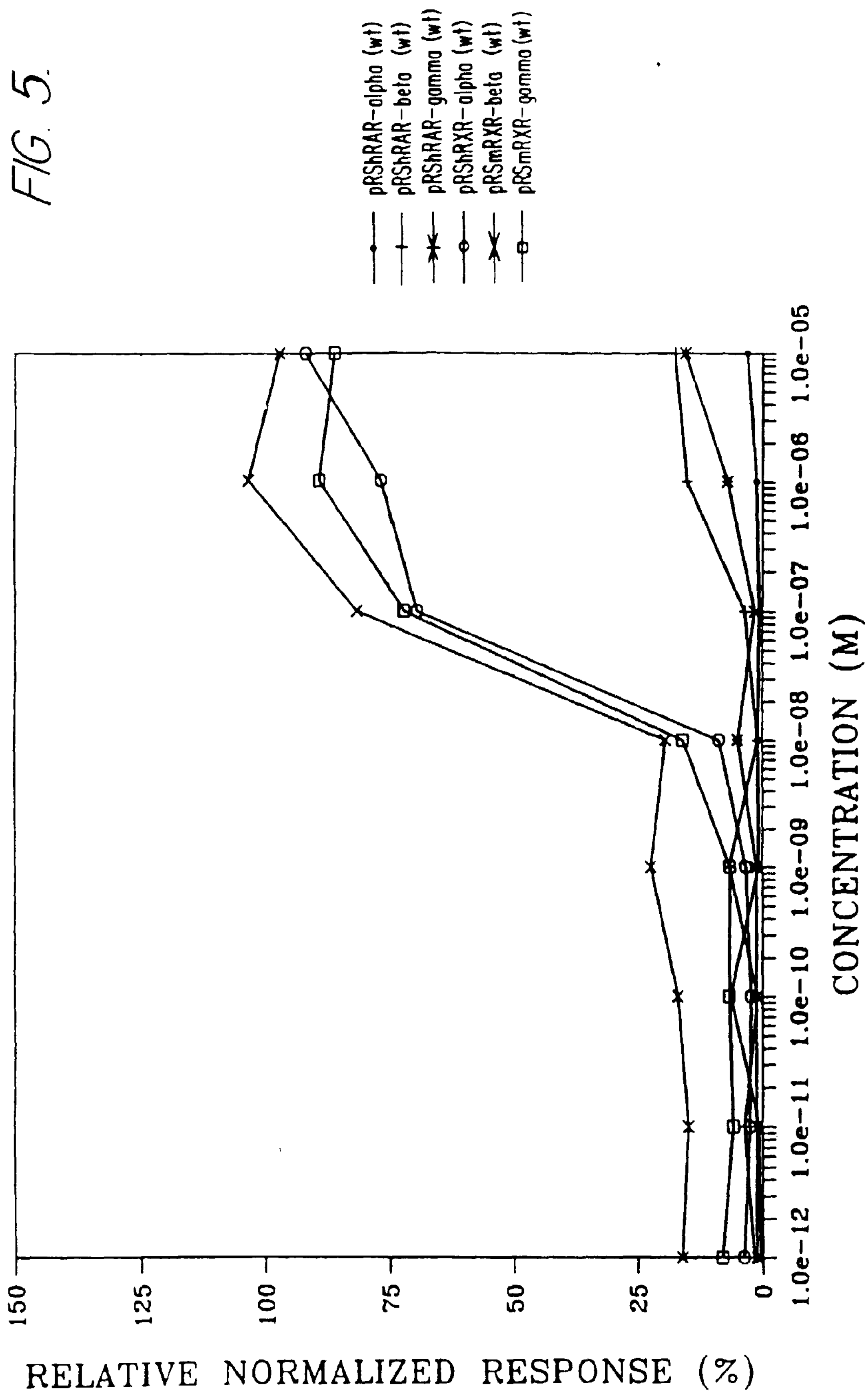
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)

FIG. 5

FIG. 6.

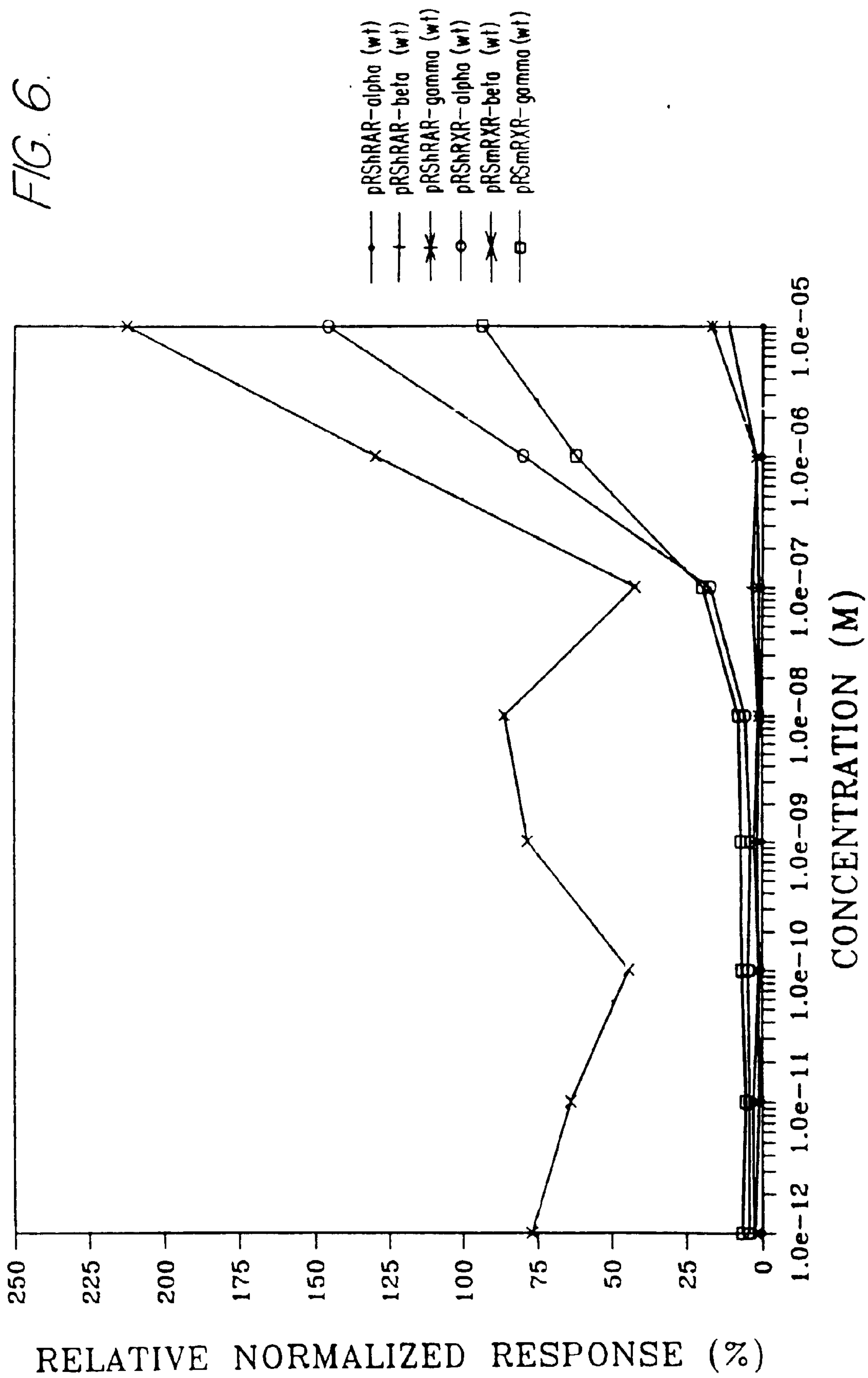

pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
RELATIVE NORMALIZED RESPONSE (%)
250
225
200
175
150
125
100
75
50
25
0
1.0e-12
1.0e-11
1.0e-10
1.0e-09
1.0e-08
1.0e-07
1.0e-06
1.0e-05
CONCENTRATION (M)

FIG. 7

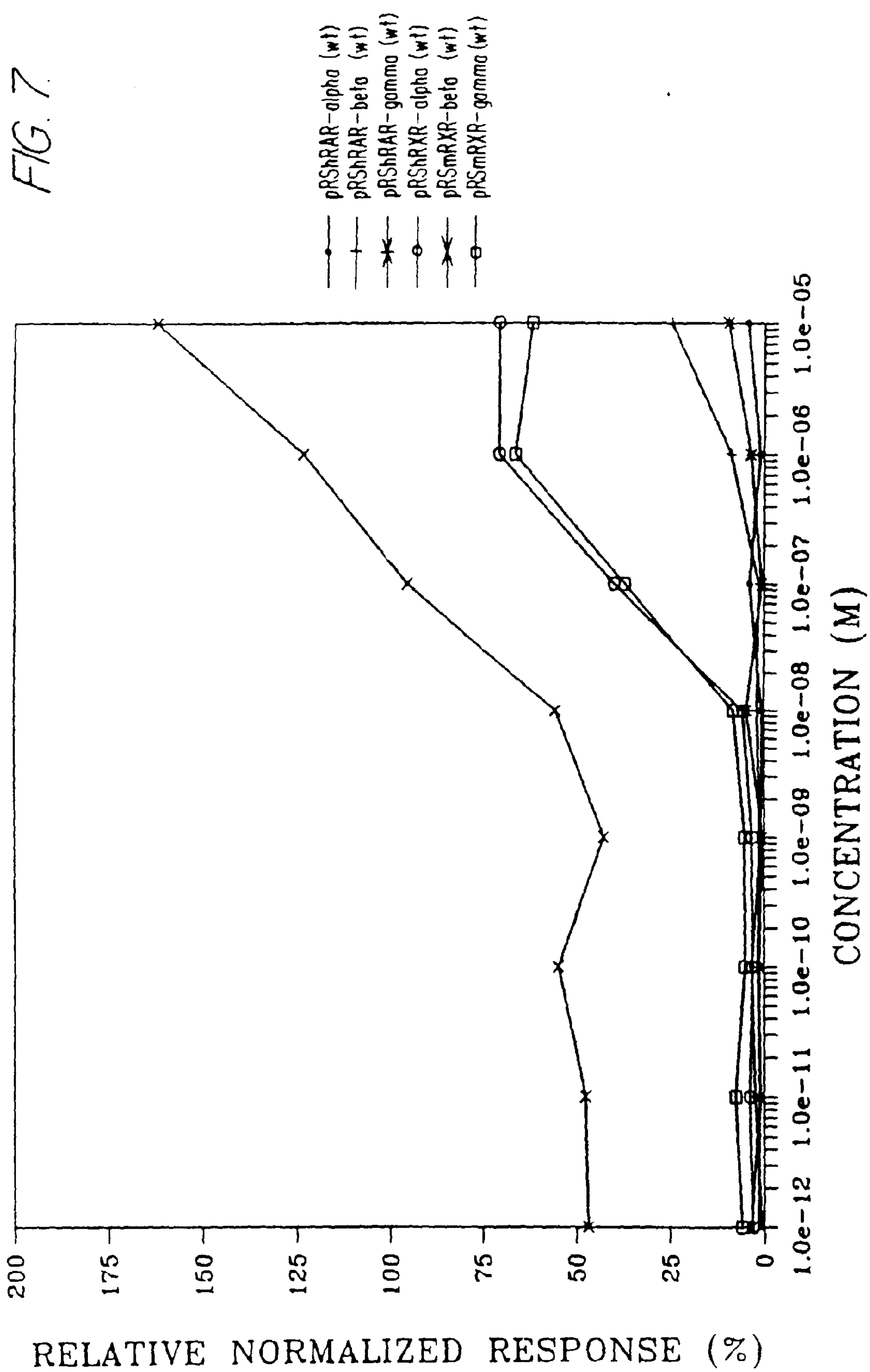
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)
200
175
150
125
100
75
50
25
0
1.0e-12
1.0e-11
1.0e-10
1.0e-09
1.0e-08
1.0e-07
1.0e-06
1.0e-05
CONCENTRATION (M)
RELATIVE NORMALIZED RESPONSE (%)

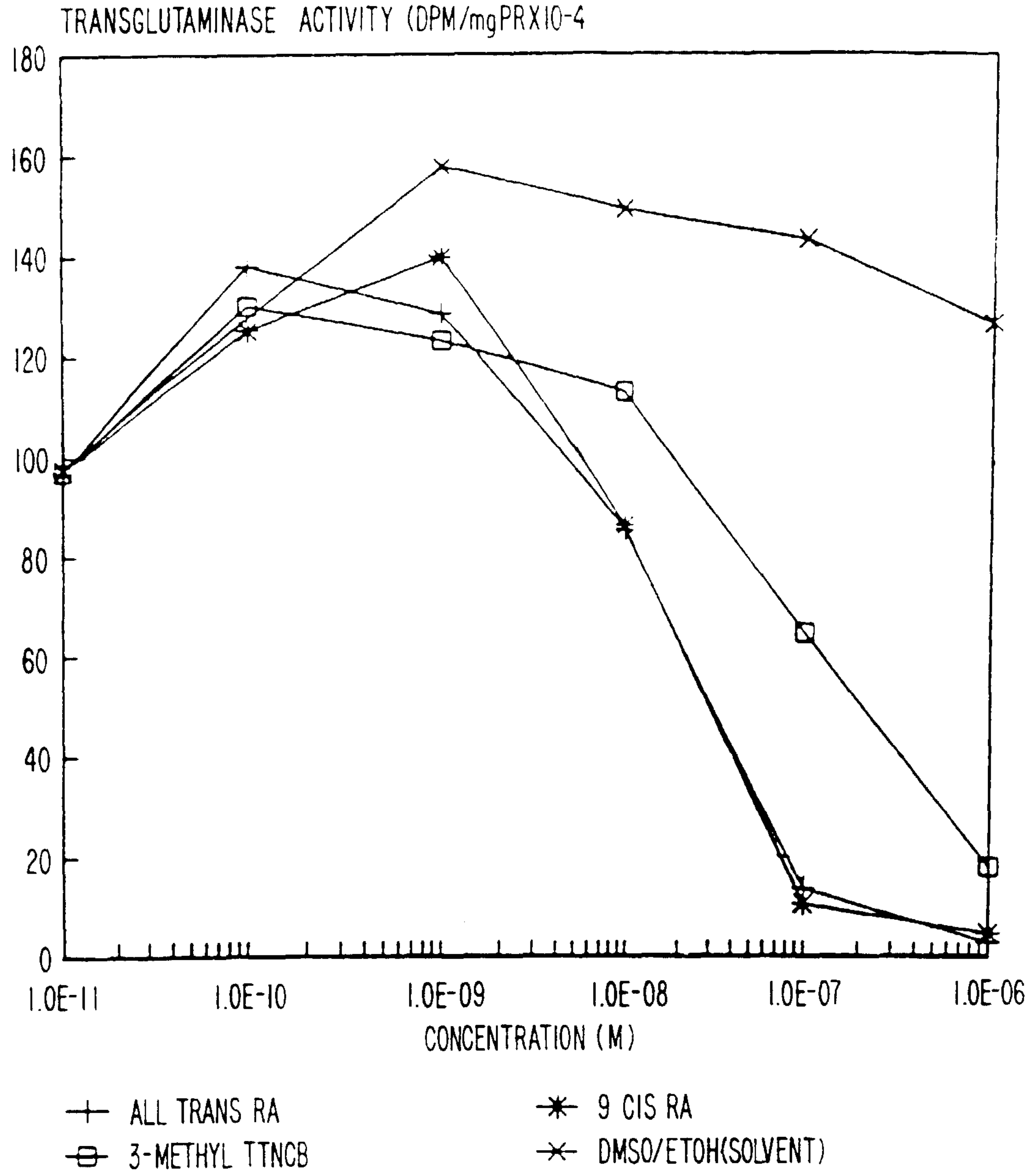
TRANSGLUTAMINASE ACTIVITY (DPM/mgPRX10-4
180
160
140
120
100
80
60
40
20
0
1.0E-11
1.0E-10
1.0E-09
1.0E-08
1.0E-07
1.0E-06
CONCENTRATION (M)
ALL TRANS RA
3-METHYL TTNCB
9 CIS RA
DMSO/ETOH(SOLVENT)

FIG. 8.

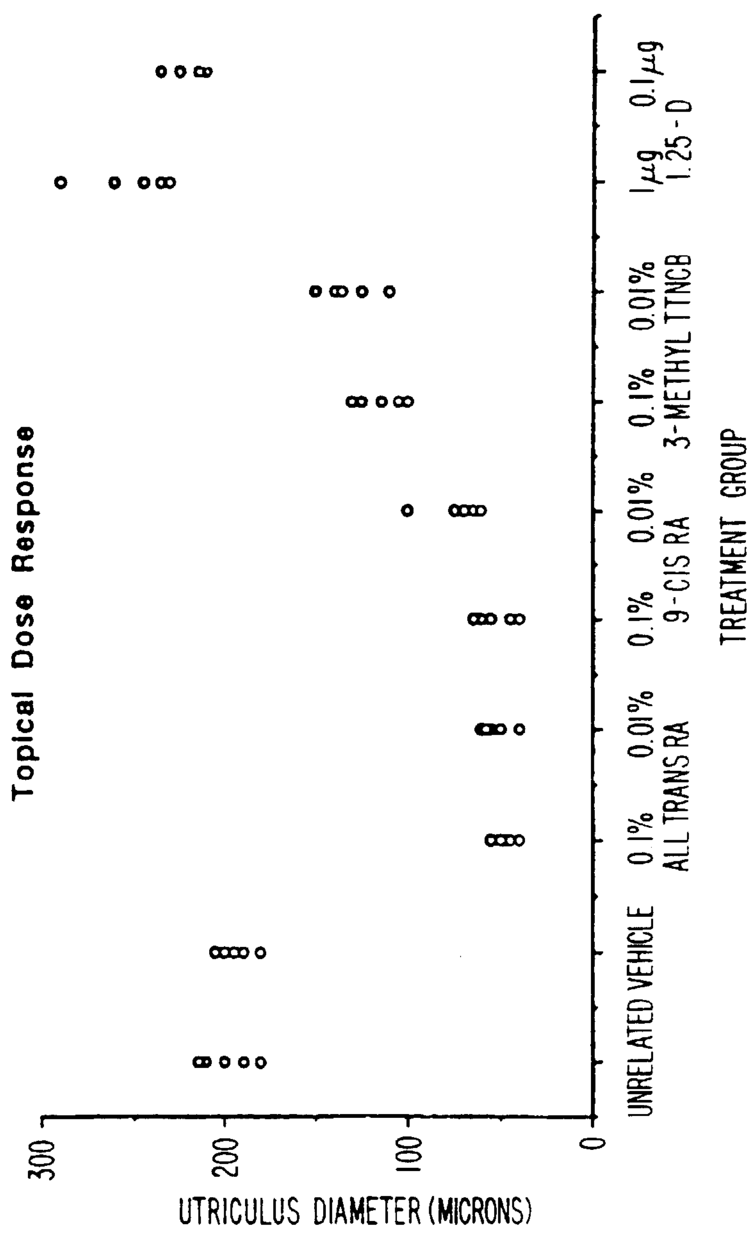
Topical Dose Response
UTRICULUS DIAMETER (MICRONS)
300
200
100
0
UNRELATED VEHICLE
0.1% 0.01%
ALL TRANS RA
0.1% 0.01%
9-CIS RA
0.1% 0.01%
3-METHYL TTNCB
1μg 0.1μg
1.25-D
TREATMENT GROUP

FIG. 9.

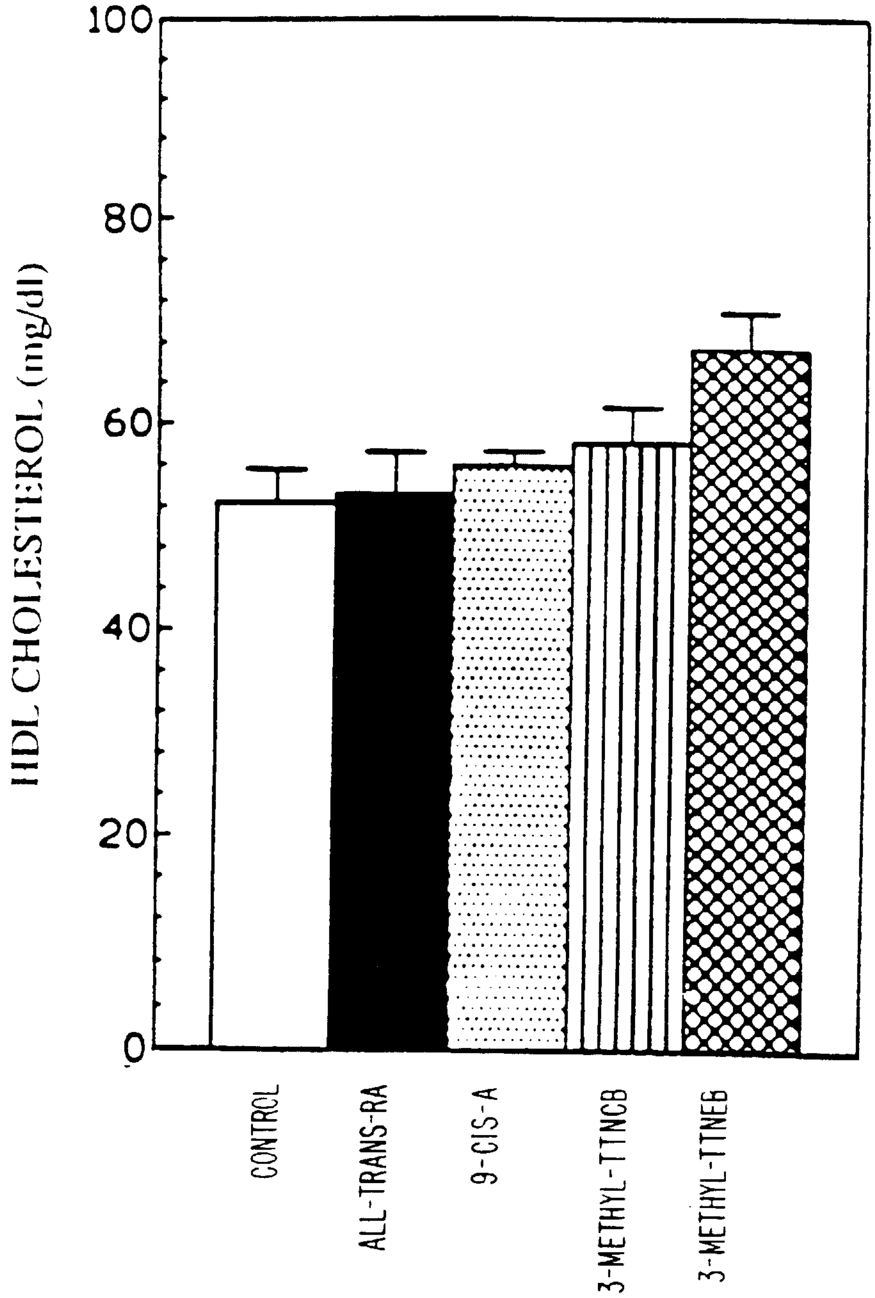
100
80
60
40
20
0
HDL CHOLESTEROL (mg/dl)
CONTROL
ALL-TRANS-RA
9-CIS-A
3-METHYL-TTNCB
3-METHYL-TTNEB

FIG. 10.

FIG. 11.

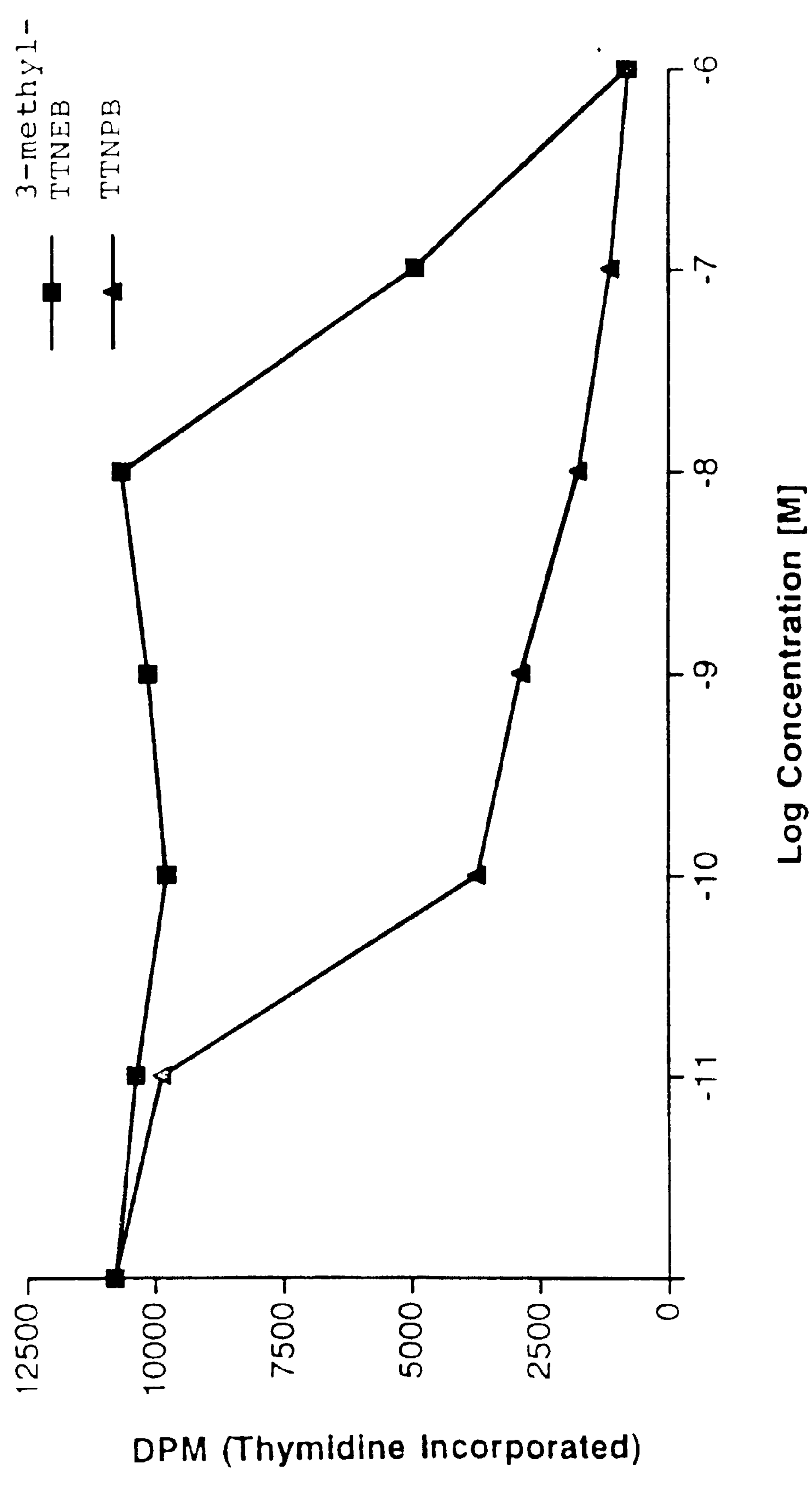
3-methyl-TTNEB
TTNPB
12500
10000
7500
5000
2500
0
-11
-10
-9
-8
-7
-6
Log Concentration [M]
DPM (Thymidine Incorporated)

FIG. 12.

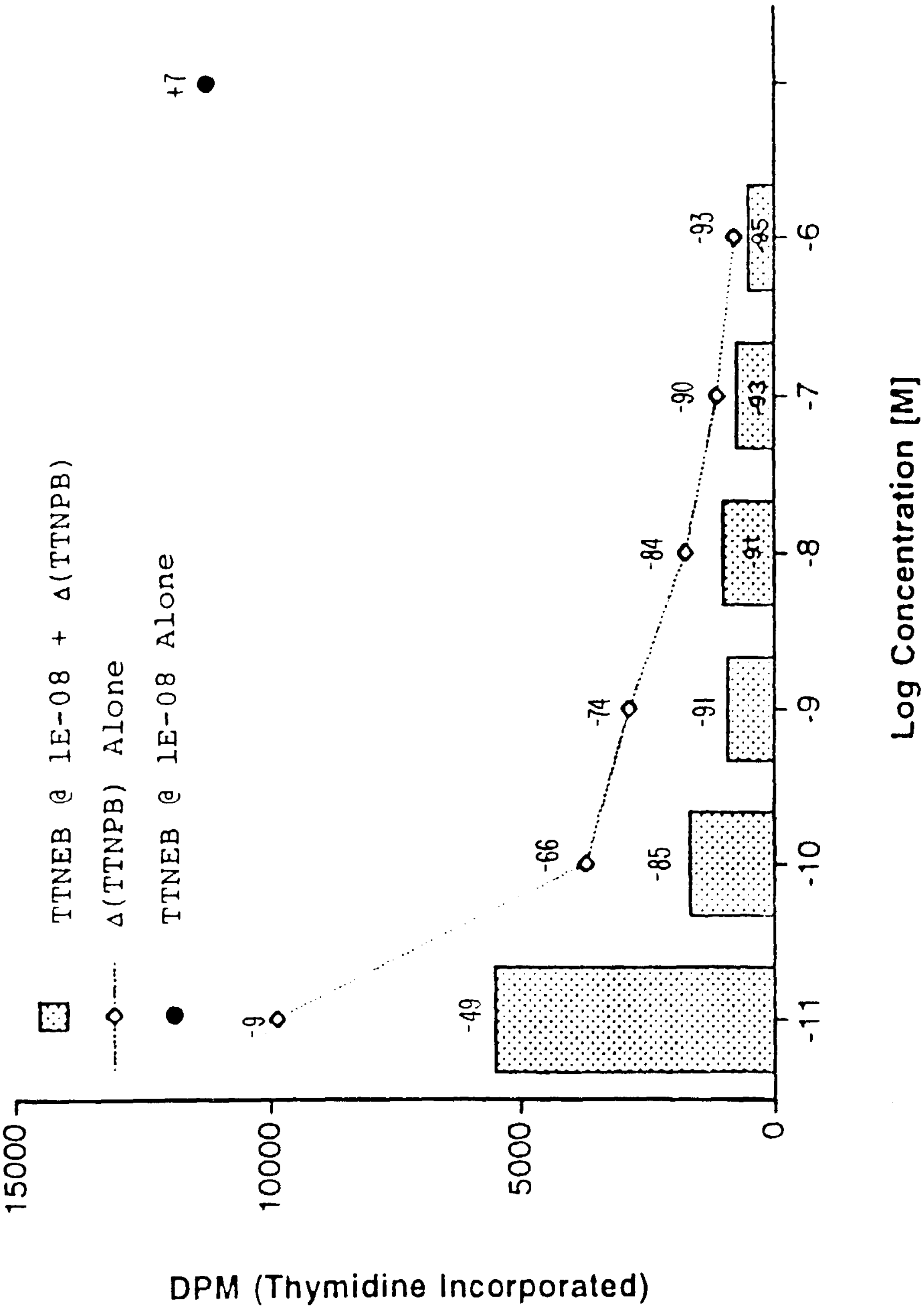

TTNEB @ 1E-08 + Δ(TTNPB)
Δ(TTNPB) Alone
TTNEB @ 1E-08 Alone
DPM (Thymidine Incorporated)
15000
10000
5000
0
Log Concentration [M]
-11
-10
-9
-8
-7
-6
-9
-66
-74
-84
-90
-93
-49
-85
-91
-91
-93
-95
+7